# EUROPÄISCHE PATENTSCHRIFT

(11) **EP 4 544 881 B1**
(45) Veröffentlichungstag und Bekanntmachung des Hinweises auf die Patenterteilung: **05.08.2026**
(21) Anmeldenummer: 23734188.8
(22) Anmeldetag: 16.06.2023
(51) Int. Cl.: H05H 1/46, B01J 19/08

(54) **VORRICHTUNG ZUR DURCHFÜHRUNG EINER CHEMISCHEN REAKTION IN EINEM PLASMA UND VERFAHREN UNTER VERWENDUNG DER VORRICHTUNG**
DEVICE FOR CARRYING OUT A CHEMICAL REACTION IN A PLASMA AND METHOD USING THE DEVICE
DISPOSITIF DE MISE EN UVRE D'UNE RÉACTION CHIMIQUE DANS UN PLASMA ET PROCÉDÉ UTILISANT LEDIT DISPOSITIF

(30) Priorität: 24.06.2022 EP 22180957; 01.07.2022 EP 22182584
(43) Veröffentlichungstag der Anmeldung: 30.04.2025
(73) Patentinhaber: FLD Technologies GmbH, 63071 Offenbach am Main (DE)
(72) Erfinder: FULDE, Marek, 63071 Offenbach (DE)
(74) Vertreter: Kudla, Karsten
(86) Internationale Anmeldenummer: PCT/EP2023/066241
(87) Internationale Veröffentlichungsnummer: WO 2023/247346

(56) Entgegenhaltungen:
- US-A1- 2004 134 890
- US-A1- 2011 298 376
- US-A1- 2018 237 709

## Beschreibung

Die Erfindung betrifft eine Vorrichtung zur Durchführung einer chemischen Reaktion in einem Plasma, wobei die Vorrichtung eine Quelle zur Erzeugung elektromagnetischer Wellen und mindestens einen Reaktor umfasst. Ferner betrifft die Erfindung ein Verfahren zur Durchführung der chemischen Reaktion unter Verwendung der Vorrichtung.

Die chemische Reaktion von gasförmigen Reaktanten findet im Plasma statt, wobei ein Feststoff beteiligt ist, der gebildet oder umgesetzt wird. Die Behandlung beziehungsweise Reaktion von Gasen in einem Plasma kann in zwei Kategorien unterteilt werden. Zum einen wird thermisches Plasma, das bei der Anwendung hochenergetischer Quellen wie einem Lichtbogen entsteht, verwendet. Das thermische Plasma ist durch Temperaturen von ca. 10.000°C gekennzeichnet und wird zwischen zwei Elektroden gebildet, die einem Verschleiß unterliegen. Thermisches Plasma wird beispielsweise zur Herstellung von Industrieruß eingesetzt.

Weiterhin ist non-thermales Plasma bekannt, das dadurch gekennzeichnet ist, dass lediglich die Elektronen des Gases einen sehr hohen energetischen Zustand erreichen, während Ionen oder Radikale einen wesentlich niedrigeren energetischen Zustand aufweisen. Das non-thermale Plasma weist einen niedrigeren Temperaturbereich von 1.000°C bis 5.000°C auf. Zur Induktion eines non-thermalen Plasmas werden häufig elektromagnetische Wellen eingesetzt. Für die Behandlung von Gasen, insbesondere für chemische Umwandlungen, wird insbesondere non-thermales Plasma eingesetzt.

Ein Beispiel dieser Technologie ist aus US2011298376 A bekannt.

M. Jasinski et al. beschreiben in "Studies of atmospheric-pressure microwave plasmas used for gas processing", Nukleonika 2012, 57(2), Seiten 241 bis 247 mehrere Verfahren zur Gasbehandlung. Dabei werden drei praxisrelevante Typen von atmosphärischen Plasmaquellen unterschieden.

Erstens wird bei der Oberflächenwellen-Entladung Plasma innerhalb eines Quarzrohrs generiert. Zweitens wird bei der düsenbasierten Lösung Plasma in einem Mikrowellenfeld gebildet, wobei das Plasmagas aus einer Düse ausströmt. Drittens strömt bei einer rohrbasierten Lösung Plasmagas aus einem zylindrischen Rohr. Bei diesen plasmagenerierenden Quellen wird ein Quarzrohr, welches das Plasma begrenzt, hohen Temperaturen ausgesetzt und muss gekühlt werden. Dadurch wird die Effektivität des Prozesses erheblich reduziert. Ferner setzen sich Feststoffpartikel wie Ruß, die an der chemischen Reaktion beteiligt sind, auf der inneren Oberfläche des Rohres ab, absorbieren die elektromagnetischen Wellen und führen zu einer Überhitzung des Rohres, die zu dessen Zerstörung führen kann. Zur Stabilisierung des Plasmas und zur Kühlung des Rohres ist eine mit der Plasmaströmung gleichläufige, wirbelförmige Gasströmung beschrieben, die eine Ablagerung der Feststoffpartikel aber nicht verhindern kann.

US 2015/0174550 beschreibt eine Methode zur Behandlung eines Reaktionsprodukts, das in Folge einer plasmabasierten Reaktion entstanden ist. Das Produkt entsteht in einer düsen- beziehungsweise rohrbasierten Plasmagas-Vorrichtung. Auch hier kommt es zur Ablagerung von Feststoff und damit zu ungünstigen thermischen Verhältnissen bei der Plasmabildung durch ungünstige Strömungsverhältnisse im Reaktor. Zwei Gasströme werden erst in Kontakt gebracht, nachdem der eine Gasstrom in ein Plasma überführt wurde. Eine effektive Vermischung von Gas und Plasma ist schwierig, da ein erheblicher Dichte- und Viskositätsunterschied vorliegt. Dies hat einen negativen Einfluss auf die chemische Reaktion.

WO 2012/147054 offenbart ein an beiden Enden geschlossenes Rohr zur Spaltung von Methan. An einem Ende des Reaktors ist ein kleineres Rohr angeordnet, aus dem ein Gas aus dem Inneren des Reaktorrohrs abfließen kann. Am selben Ende des Rohrs wird dem Reaktor das Reaktionsgas zugeführt. Die Spaltung von Methan wird in einem Plasma durchgeführt, das durch elektromagnetische Wellen indiziert wird. Die Reaktanten werden vor der Zuführung in den Reaktor gemischt, so dass eine separate Einstellung der Temperatur der einzelnen Reaktionsteilnehmer nicht möglich ist. Die Reaktion des Gemischs der Reaktanten startet beim Passieren eines Mikrowellenfensters, was zur Rußbildung am Fenster und damit zu einer Überhitzung des Fensters führt.

Eine Aufgabe der Erfindung besteht darin, eine Vorrichtung und ein Verfahren bereitzustellen, wobei die Nachteile aus dem Stand der Technik vermieden werden. Die Ablagerung von Feststoffpartikeln an der Reaktorwand und insbesondere am Fenster zur Durchleitung elektromagnetischer Wellen soll reduziert oder verhindert werden. Weiterhin soll die Mischung verschiedener Gasströme innerhalb des Reaktors ermöglicht beziehungsweise verbessert werden, so dass ein homogenes Reaktionsgemisch und ein stabiles Plasma entstehen.

### Offenbarung der Erfindung

Es wird eine Vorrichtung zur Durchführung einer chemischen Reaktion in einem Plasma vorgeschlagen. Diese Vorrichtung umfasst eine Quelle zur Erzeugung elektromagnetischer Wellen, mindestens einen ersten Reaktor, mindestens ein Verbindungsstück und einen zweiten Reaktor, wobei der erste Reaktor, das Verbindungsstück und der zweite Reaktor jeweils als Rohre ausgeführt sind, das Verbindungstück einen kleineren Durchmesser aufweist als der erste Reaktor und die Quelle zur Erzeugung elektromagnetischer Wellen an dem ersten Reaktor angeordnet ist, wobei der erste Reaktor eine erste Mantelfläche und erste Endflächen aufweist und der zweite Reaktor eine zweite Mantelfläche und zweite Endflächen aufweist und der zweite Reaktor ein zumindest teilweise in dem zweiten Reaktor angeordnetes Innenrohr besitzt, so dass ein innerer Gasraum und ein äußerer Gasraum gebildet werden, die an der dem Verbindungsstück näherliegenden zweiten Endfläche des zweiten Reaktors voneinander getrennt sind, wobei das Verbindungsstück den ersten Reaktor und den zweiten Reaktor fluidisch miteinander verbindet und das Verbindungsstück an einer der ersten Endflächen aus dem ersten Reaktor austritt und an der zweiten Mantelfläche in den äußeren Gasraum des zweiten Reaktors mündet, insbesondere in tangentialer Ausrichtung, und wobei die erste Mantelfläche des ersten Reaktors einen ersten Abschnitt, mit mindestens einem Einlass zur Zufuhr eines ersten Eingangsgases, einen zweiten Abschnitt mit mindestens einem Einlass zur Zufuhr eines zweiten Eingangsgases und ein für die elektromagnetischen Wellen durchlässiges Fenster, das insbesondere aus Quarz, Aluminiumoxid, Bornitrid oder Polytetrafluorethylen hergestellt ist, aufweist, wobei der Einlass zur Zufuhr des ersten Eingangsgases und der Einlass zur Zufuhr des zweiten Eingangsgases tangential zur ersten Mantelfläche des ersten Reaktors ausgerichtet sind und der erste Abschnitt und der zweite Abschnitt axial versetzt angeordnet sind, wobei der zweite Abschnitt näher als der erste Abschnitt an der ersten Endfläche liegt, aus der das Verbindungsstück aus dem ersten Reaktor austritt, und das Fenster zwischen dem ersten Abschnitt und dem zweiten Abschnitt angeordnet ist, und der zweite Reaktor, insbesondere an einer der zweiten Endflächen, einen Auslass zur Abfuhr eines Produktstroms aufweist und die zweite Mantelfläche des zweiten Reaktors einen dritten Abschnitt mit mindestens einem Einlass zur Zufuhr eines dritten Eingangsgases aufweist und der dritte Abschnitt insbesondere an einer dem Verbindungsstück entfernteren der zweiten Endflächen des zweiten Reaktors angeordnet ist.

Ferner wird ein Verfahren zur Durchführung der chemischen Reaktion unter Verwendung der erfindungsgemäßen Vorrichtung vorgeschlagen, wobei ein Feststoff, insbesondere Kohlenstoff, beteiligt ist, insbesondere gebildet oder umgesetzt wird, und die chemische Reaktion bevorzugt ausgewählt ist aus der Gruppe bestehend aus Pyrolyse von Kohlenwasserstoffen, insbesondere von Methan, Herstellung von Acetylen, Reformierung von Kohlenwasserstoffen, insbesondere Methan, Pyrolyse von Schwefelwasserstoff, Pyrolyse von Ammoniak und Hydrovergasung von Kohlenstoff.

Der erste Reaktor und der zweite Reaktor bilden eine zweistufige Reaktorkaskade. Der erste Reaktor und der zweite Reaktor, also die beiden Stufen, sind durch das Verbindungsstück miteinander verbunden.

Durch den Einlass am ersten Abschnitt beziehungsweise am zweiten Abschnitt der ersten Mantelfläche entstehen nah an der ersten Mantelfläche, also der Innenwand des Rohrs, zwei wirbelartige Strömungen, die sich aufeinander zu bewegen. Die Mantelflächen können jeweils auch als Mantel bezeichnet werden, der insbesondere im Verhältnis zum Reaktordurchmesser eine nur geringe Dicke aufweist. **In** der Nähe des von dem Verbindungsstück entfernteren Ende des ersten Reaktors entsteht eine Mischzone, also am ersten Abschnitt, in welcher das erste Eingangsgas und das zweite Eingangsgas intensiv gemischt werden und einen inneren Wirbel formen, der sich in Richtung des Verbindungsstücks bewegt.

An dem Fenster, das zwischen dem ersten Abschnitt und dem zweiten Abschnitt angeordnet ist, zum Beispiel in der Mitte in axialer Richtung des ersten Reaktors, wird Plasma injiziert. Unter einem Plasma wird ein Gemisch von Elektronen, Ionen und Radikalen verstanden. Der innere Wirbel durchströmt die Plasmazone und erfährt eine intensive Energie-Einkopplung, was zur Initiierung chemischer Reaktionen in der Gasphase führt.

Durch die tangentiale und gegebenenfalls an verschiedenen Seiten des Fensters angeordnete Zuführung des ersten Eingangsgases und des zweiten Eingangsgases in den ersten Reaktor werden zwei sich aufeinander zu bewegende Wirbelströmungen ausgebildet, die insbesondere außerhalb des Fensters aufeinandertreffen und zur Vermischung des ersten Eingangsgases mit dem zweiten Eingangsgas führen, wobei eine Eingangsgasmischung entsteht. Die Eingangsgasmischung strömt an dem Fenster des ersten Reaktors vorbei und wird durch die eingehenden elektromagnetischen Wellen in ein Plasma überführt, das dann durch das Verbindungsstück den ersten Reaktor verlässt und in den zweiten Reaktor eintritt. Das Plasma wird dazu von dem Innenraum des ersten Reaktors in das Verbindungsstück geleitet.

Durch die Wirbelströmungen wird ferner das Plasma in dem ersten Reaktor stabilisiert und durch die tangentiale Gasströmung an der Reaktorwand, also der ersten Mantelfläche, wird eine Ablagerung von Feststoff an der Mantelfläche und/oder am Fenster vermieden.

Durch die erfindungsgemäße Zuführung werden das erste Eingangsgas und das zweite Eingangsgas miteinander vermischt, wenn sich beide Gase noch in einem gasförmigen Zustand befinden. Erst anschließend werden die in Mischung befindlichen Gase gemeinsam in ein Plasma überführt, so dass die Dichte- und Viskositätsunterschiede zwischen dem gasförmigen Zustand und dem Plasmazustand einer Vermischung nicht entgegenstehen. Die Quelle zur Erzeugung elektromagnetischer Wellen dient der Plasmaerzeugung. Das erste Eingangsgas ist bevorzugt ein Reaktionsgas. Das zweite Eingangsgas ist bevorzugt ein zusätzliches Gas, das insbesondere zur Förderung der Plasma-Bildung eingesetzt wird.

Die für den Ablauf der chemischen Reaktion notwendige Energie wird am Fenster, also in einer Plasmazone, dem Eingangsgasgemisch in Form der elektromagnetischen Wellen zugeführt. Das zweite Eingangsgas, das zum Beispiel inerte Gase enthält, kann zur Verstärkung der Plasmabildung dienen, insbesondere in einem Fall, in dem das eigentliche Reaktionsgas weniger empfindlich auf elektromagnetische Wellen reagiert und weniger leicht aus sich heraus ein Plasma bildet. Die inerten Gase können auch als Energieträger verstanden werden, da sie im Plasma einen hohen energetischen Zustand erreichen und anschließend Energie an das eigentliche Reaktionsgas abgeben können.

Das Verbindungsstück wird insbesondere tangential in oder an der zweiten Mantelfläche des zweiten Reaktors, also der zweiten Stufe der Kaskade angeordnet, so dass auch in dem zweiten Reaktor eine wirbelartige Strömung erzeugt wird. Bevorzugt wird das gebildete Plasma tangential in den zweiten Reaktor geführt. Eine tangentiale Zuführung des Plasmas in den äußeren Gasraum des zweiten Reaktors führt auch hier zur Ausbildung einer Wirbelströmung, die sich durch den zweiten Reaktor erstreckt, gegebenenfalls in Kontakt mit dem dritten Eingangsgas, insbesondere mit einem dritten Eingangsgasstrom, kommt und dann den zweiten Reaktor an einer der zweiten Endflächen verlässt. Die ersten Endflächen und die zweiten Endflächen können auch als Grundflächen bezeichnet werden.

Die tangentiale Ausrichtung ist insbesondere bezüglich der ersten Mantelfläche des ersten Reaktors beziehungsweise der zweiten Mantelfläche des zweiten Reaktors gegeben, die insbesondere die innere Oberfläche der Reaktorwand bilden.

Die Einlässe zur Zuführung des ersten, zweiten beziehungsweise dritten Eingangsgases können auch als Gaszugabe-Vorrichtungen bezeichnet werden, die insbesondere in Form von tangential ausgerichteten Bohrungen durch die Außenwand, also die jeweilige Mantelfläche ausgeführt sein können. Die tangentiale Ausrichtung ist insbesondere auf den Umfang des ersten Reaktors beziehungsweise des zweiten Reaktors bezogen.

Die Einlässe werden insbesondere jeweils durch, bevorzugt gerade, Bohrungen gebildet, wobei eine Bohrungsrichtung und die erste Mantelfläche beziehungsweise die zweite Mantelfläche bevorzugt einen Winkel von weniger als 45°, weiter bevorzugt weniger als 30° und noch weiter bevorzugt weniger als 20°, insbesondere weniger als 10° aufweisen. Dadurch strömt das erste Eingangsgas, das zweite Eingangsgas beziehungsweise das dritte Eingangsgas zunächst im Wesentlichen parallel zu der ersten Mantelfläche des ersten Reaktors beziehungsweise der zweiten Mantelfläche des zweiten Reaktors und in Umfangsrichtung, so dass sich eine wandnahe Wirbelströmung innerhalb des ersten Reaktors beziehungsweise innerhalb des zweiten Reaktors ausbildet.

Durch die Wirbelströmungen werden Strömungsverhältnisse in den Reaktoren ausgebildet, die denen eines idealen Rohrreaktors ähneln.

Der erste Reaktor, der zweite Reaktor und/oder das Verbindungstück sind bevorzugt jeweils als zylindrische Rohre ausgeführt. Die Querschnittsfläche des ersten Reaktors, des zweiten Reaktors und/oder des Verbindungstücks ist bevorzugt kreisförmig, elliptisch oder vieleckig, wie rechteckig. Insbesondere ist die Querschnittsfläche des ersten Reaktors, des zweiten Reaktors und/oder des Verbindungstücks kreisförmig.

Das Verbindungsstück weist bevorzugt einen um mindestens 30 % kleineren Durchmesser als ein Reaktordurchmesser des ersten Reaktors auf, bezogen auf den Reaktordurchmesser des ersten Reaktors. Das Innenrohr des zweiten Reaktors weist bevorzugt einen um mindestens 30 % kleineren Durchmesser als ein Reaktordurchmesser des zweiten Reaktors auf, bezogen auf den Reaktordurchmesser des zweiten Reaktors.

Der äußere Gasraum ist insbesondere durch eine Innenwand des zweiten Reaktors, also die zweite Mantelfläche, eine Außenwand des Innenrohrs und eine der zweiten Endflächen des zweiten Reaktors begrenzt. Der innere Gasraum ist insbesondere durch eine Innenwand des Innenrohrs und gegebenenfalls durch eine Innenrohrseitenfläche begrenzt.

Der mindestens eine Einlass zur Zufuhr des ersten Eingangsgases und der mindestens eine Einlass zur Zufuhr des zweiten Eingangsgases liegen insbesondere an gegenüberliegenden Seiten des Fensters, so dass der erste Abschnitt und der zweite Abschnitt in axialer Richtung des ersten Reaktors durch das Fenster voneinander getrennt sind.

Der zweite Abschnitt und damit der Einlass des zweiten Eingangsgases ist auf derselben Seite des Fensters angeordnet wie das Verbindungsstück. So strömt das zweite Eingangsgas in Form eines Wirbels zunächst am Fenster vorbei in Richtung des ersten Abschnitts mit dem Einlass zur Zufuhr des ersten Eingangsgases und wird mit diesem vermischt, wobei das Eingangsgasgemisch gebildet wird. Das Eingangsgasgemisch strömt an dem Fenster vorbei in das Verbindungsstück hinein und wird am Fenster in ein Plasma überführt, das räumlich durch die das Plasma umgebende Wirbelströmung des zweiten Eingangsgases stabilisiert wird.

Bevorzugt bilden das erste Eingangsgas und das zweite Eingangsgas sich aufeinander zu bewegende Wirbelströmungen in dem ersten Reaktor aus. Weiterhin strömt das zweite Eingangsgas bevorzugt sich auf das Plasma zu bewegend. Bevorzugt ist die Gesamtströmungsrichtung des zweiten Eingangsgases, insbesondere bei Eintritt in den Reaktor, der Gesamtströmungsrichtung des Plasmas entgegengesetzt. Das Plasma verlässt den ersten Reaktor durch das Verbindungsstück und wird in den zweiten Reaktor überführt.

**In** dem zweiten Reaktor wird bevorzugt eine weitere Wirbelströmung, insbesondere um das Innenrohr herum, ausgebildet. Je nach Anordnung des Innenrohrs in dem zweiten Reaktor verlässt das gebildete Produktgas der chemischen Reaktion den zweiten Reaktor in der Nähe des Verbindungsstücks oder an der von dem Verbindungstück entfernteren zweiten Endfläche. Ist der Auslass des Produktgasstroms auf der Seite des Verbindungsstücks an dem zweiten Reaktor angeordnet, bildet sich noch eine weitere Wirbelströmung im inneren Gasraum, also innerhalb des Innenrohrs, in Richtung des Auslasses aus. In dieser Ausführungsform bewegt sich die Wirbelströmung im inneren Gasraum auf die Wirbelströmung im äußeren Gasraum zu.

Der zweite Reaktor ist insbesondere an beiden zweiten Endflächen geschlossen, mit Ausnahme des Auslasses des Produktstroms. Bevorzugt wird das Innenrohr durch eine der zweiten Endflächen hindurchgeführt, insbesondere durch die Endfläche, die dem Verbindungsstück am nächsten ist. An dem gegenüberliegenden Ende ist bevorzugt der mindestens eine Einlass des dritten Eingangsgases angeordnet. Auch das dritte Eingangsgas wird bevorzugt tangential in den zweiten Reaktor eingeleitet.

Das Innenrohr ist bevorzugt an der zweiten Endfläche des zweiten Reaktors befestigt, die dem Verbindungsstück am nächsten liegt. Der Auslass aus dem zweiten Reaktor kann an dieser zweiten Endfläche oder an der gegenüberliegenden zweiten Endfläche des zweiten Reaktors angeordnet sein. **In** einer Ausführungsform weist das Innenrohr ein offenes Ende und ein geschlossenes Ende auf, wobei das offene Ende an dem Auslass des zweiten Reaktors angeordnet ist. **In** einer zweiten Ausführungsform weist das Innenrohr zwei offene Enden auf, wobei eines der zwei offenen Enden den Auslass des zweiten Reaktors bildet. Eine der zweiten Endflächen kann offen sein und den Auslass bilden.

**Der zweite** Reaktor kann ein konisches Ende aufweisen, wobei der Auslass bevorzugt an einem konischen Ende angeordnet ist und das konische Ende weiter bevorzugt das von dem Verbindungsstück entferntere Ende des zweiten Reaktors bildet. Insbesondere kann eine der zweiten Endflächen konisch ausgebildet sein. Hierbei weist das Innenrohr bevorzugt ein offenes Ende und ein geschlossenes Ende auf, wobei das offene Ende bevorzugt an dem konischen Ende des zweiten Reaktors angeordnet ist.

Der zweite Reaktor kann mindestens einen Einlass zur Zufuhr eines dritten Eingangsgases aufweisen, der bevorzugt an der von dem Verbindungstück entfernteren zweiten Endfläche des zweiten Reaktors angeordnet ist. Das Produktgas kann mit dem dritten Eingangsgas gemischt werden. Das dritte Eingangsgas weist insbesondere eine geringere Temperatur in einem Bereich von 273 K bis 1000 K auf. Damit dient das dritte Eingangsgas zur Abkühlung des Plasmas beziehungsweise des Produktgases.

Der Durchmesser des Verbindungsstücks wird bevorzugt so gewählt, dass die Ausbreitung der elektromagnetischen Wellen in dem Verbindungsstück nicht mehr möglich ist. Hierdurch wird die Wirkung des elektromagnetischen Feldes auf den ersten Reaktor beschränkt. Die in dem ersten Reaktor initiierte Reaktion wird bevorzugt im Verbindungsstück fortgesetzt.

Das Verbindungsstück dient insbesondere als Barriere für die elektromagnetischen Wellen, die sich somit nicht in das Verbindungsstück und den zweiten Reaktor ausbreiten. Bevorzugt weist das Verbindungsstück einen konstanten Durchmesser auf. Eine Frequenz f des elektromagnetischen Felds und der Durchmesser D des Verbindungsstücks genügen bevorzugt der Bedingung D*f < 175,7, wobei der Durchmesser D in mm und die Frequenz f in GHz angegeben sind. Bevorzugt weisen der erste Reaktor und/oder der zweite Reaktor jeweils einen konstanten Reaktordurchmesser auf. Der Reaktordurchmesser des zweiten Reaktors wird bevorzugt in Abhängigkeit von der Reaktionskinetik gewählt. Bevorzugt ist der Reaktordurchmesser des zweiten Reaktors gleich dem oder größer als, mehr bevorzugt größer als, der Durchmesser des Verbindungsstücks. Der größere Reaktordurchmesser des zweiten Reaktors dient der Ausbildung der Wirbelströmung, die auch als Drallströmung bezeichnet werden kann, im zweiten Reaktor.

Bevorzugt wird eine Zwei-Phasen-Strömung durch das Verbindungsstück geführt. Die Fließgeschwindigkeit im Verbindungsstück beträgt bevorzugt mindestens 20 m/s.

Der erste Reaktor und das Verbindungsstück sind bevorzugt parallel zueinander angeordnet. Der zweite Reaktor und das Innenrohr sind bevorzugt parallel zueinander angeordnet. Weiter bevorzugt sind der erste Reaktor und das Verbindungsstück koaxial angeordnet. Weiter bevorzugt sind der zweite Reaktor und das Innenrohr koaxial angeordnet.

Das Innenrohr erstreckt sich bevorzugt von der dem Verbindungsstück näherliegenden zweiten Endfläche des zweiten Reaktors bis zu einer Länge in einem Bereich von 50 % bis 70 % einer Gesamtlänge des zweiten Reaktors.

Das Verbindungsstück, das auch als Plasmaleiter bezeichnet werden kann, weist bevorzugt eine Länge in einem Bereich von 30 bis 1000 mm auf. Ferner beträgt die Länge des Verbindungsstücks bevorzugt das 2-fache bis 50-fache des Reaktordurchmessers des ersten Reaktors, mehr bevorzugt das 5-fache bis 25-fache, insbesondere das 5-fache bis 10-fache. Bevorzugt wird die Länge des Verbindungstücks so eingestellt, dass das Plasma im Verbindungsstück oder bei Eintritt in den zweiten Reaktor erlischt. Damit finden die anschließenden Reaktionen im zweiten Reaktor statt.

Die Quelle zur Erzeugung elektrischer Wellen umfasst bevorzugt einen Wellenkanal, der zum Fenster führt, so dass die elektromagnetischen Wellen in den ersten Reaktor eindringen können. Ein Abstand zwischen einem, dem Verbindungsstück zugewandten Ende des Fensters und der ersten Endfläche, an der das Verbindungsstück angeordnet ist, beträgt bevorzugt 20 bis 50 %, weiter bevorzugt 20 bis 40 % und insbesondere bevorzugt 20 bis 25 % einer Gesamtlänge des ersten Reaktors, insbesondere in axialer Richtung.

Das Fenster ist bevorzugt über den gesamten Umfang des ersten Reaktors ausgebildet. Bevorzugt nimmt das Fenster 5 % bis 30 % einer Gesamtfläche der ersten Mantelfläche des ersten Reaktors ein.

Unter einem Material, das für die elektromagnetischen Wellen durchlässig ist, wird insbesondere verstanden, dass das Material eine niedrige Dielektrizitätskonstante, also niedrige relative Permittivität, aufweist. Bevorzugt als Material, das für die elektromagnetischen Wellen durchlässig ist, sind Materialien mit einer Dielektrizitätskonstanten von kleiner 10, insbesondere Polytetrafluorethylen (PTFE), Bornitrid, Quarz, Siliziumdioxid und/oder Aluminiumoxid.

Das Verbindungsstück ist bevorzugt gerade. Bevorzugt sind der erste Reaktor und der zweite Reaktor in einem Winkel in einem Bereich von 60° bis 120°, insbesondere von 80° bis 100°, zueinander angeordnet, bezogen auf die Mittelachsen des ersten Reaktors bzw. des zweiten Reaktors.

Bevorzugt erstreckt sich das Verbindungsstück in den ersten Reaktor über den mindestens einen Einlass des zweiten Abschnitts bis maximal zu dem Fenster. Das Verbindungsstück ragt also bevorzugt in den ersten Reaktor hinein, insbesondere ausgehend von einer der ersten Endflächen. Bevorzugt bildet das erste Eingangsgas eine Wirbelströmung zunächst um das Verbindungsstück herum. Das Verbindungsstück erstreckt sich nicht weiter als bis zum Fenster, um eine Abdeckung der eingehenden elektromagnetischen Wellen zu vermeiden. Der Fortsatz des Verbindungsstücks innerhalb des ersten Reaktors dient zur Aufnahme und Stabilisierung, also zur räumlichen Einengung, des Plasmas, so dass dieses mindestens bis zum Eintritt in den zweiten Reaktor weiterbesteht.

Bevorzugt weisen der erste Abschnitt und/oder der zweite Abschnitt jeweils mindestens zwei Einlässe auf. Die mindestens zwei Einlässe sind weiter bevorzugt gegenüberliegend angeordnet, bezogen auf eine Mittelachse des ersten Reaktors. Dabei wird das erste Eingangsgas und/oder das zweite Eingangsgas bevorzugt jeweils auf die mindestens zwei Einlässe aufgeteilt. Durch Zufuhr des ersten Eingangsgases beziehungsweise des zweiten Eingangsgases an mindestens zwei Positionen des Umfangs des ersten Reaktors wird die Ausbildung der Wirbelströmungen gefördert. Die mindestens zwei Einlässe des ersten Abschnitts beziehungsweise des zweiten Abschnitts sind bevorzugt auf der gleichen axialen Höhe des ersten Reaktors angeordnet.

Die Vorrichtung kann mindestens zwei, insbesondere vier, sechs, acht oder zwölf oder mehr erste Reaktoren mit jeweils einem Verbindungsstück aufweisen. Weiter bevorzugt münden die Verbindungsstücke jeweils tangential in den äußeren Gasraum des zweiten Reaktors. Insbesondere weist jeder erste Reaktor eine Quelle zur Erzeugung elektromagnetischer Wellen auf. **In** den ersten Reaktoren wird bevorzugt jeweils ein Plasma erzeugt, welche in dem zweiten Reaktor zusammengeführt werden und ein gemeinsames Produktgas bilden.

Das Verbindungsstück und/oder das Innenrohr können mit einer Vibrationsvorrichtung ausgerüstet sein. Mit der, insbesondere elektrischen, Vibrationsvorrichtung kann das Verbindungsstück und/oder das Innenrohr kontinuierlich oder phasenweise in Schwingung versetzt werden. Durch die Vibrationsvorrichtung können abgelagerte Feststoffpartikel entfernt bzw. die Ablagerung zusätzlich verhindert werden.

Bevorzugt sind zumindest Teile der ersten Mantelfläche des ersten Reaktors und/oder das Verbindungsstück mit einer Heizvorrichtung ausgerüstet. Mit der Heizvorrichtung kann die Temperatur lokal gezielt eingestellt werden. Durch die Heizvorrichtung an der ersten Mantelfläche, insbesondere am zweiten Abschnitt, kann insbesondere das zweite Eingangsgas vorgewärmt werden. Das Verbindungsstück kann beheizt werden, um die chemische Reaktion zu begünstigen.

Bevorzugt ist der erste Reaktor aus einem für die elektromagnetischen Wellen undurchlässigen Material, insbesondere Stahl, Bronze oder Aluminium hergestellt. Der zweite Reaktor, insbesondere einschließlich dem Innenrohr, und/oder das Verbindungsstück sind bevorzugt aus einem hochtemperaturstabilen Material, insbesondere Graphit, Quarzglas oder einem Metall wie Wolfram oder Molybdän, hergestellt. Das hochtemperaturstabile Material ist insbesondere bis zu einer Temperatur von 2000°C stabil.

Der erste Reaktor stellt bevorzugt einen geschlossenen Behälter mit Ausnahme der Durchführung des Verbindungsstücks dar. Insbesondere sind bevorzugt beide erste Endflächen des ersten Reaktors geschlossen, wobei eine der ersten Endflächen das Verbindungsstück aufweist.

Die chemische Reaktion ist bevorzugt eine endotherme Reaktion, die also eine Energiezufuhr erfordert. Als Energiequelle dient das Plasma, das mittels der elektromagnetischen Wellen im ersten Reaktor erzeugt wird. Die chemische Reaktion findet bevorzugt in der Plasma- und/oder Gasphase statt. Die Plasma- und/oder Gasphase, beziehungsweise die Reaktanten, können Gase und/oder dampfförmige Komponenten umfassen. An der chemischen Reaktion ist bevorzugt ein Feststoff beteiligt. Als Reaktanten, die auch als Ausgangstoffe oder Edukte bezeichnet werden können, werden insbesondere Stoffe verstanden, die dem ersten Reaktor und/oder dem zweiten Reaktor, bevorzugt dem ersten Reaktor, zugeführt und dort chemisch umgewandelt werden. Der Feststoff wird insbesondere gebildet oder als Reaktant eingesetzt, wird also umgesetzt. Der Feststoff enthält bevorzugt Kohlenstoff und ist weiter bevorzugt Kohlenstoff, also besteht aus Kohlenstoff. Der Kohlenstoff ist insbesondere atomarer Kohlenstoff, der auch als Ruß, Industrieruß oder Carbon Black bezeichnet wird.

Die chemische Reaktion ist insbesondere ausgewählt aus der Gruppe bestehend aus Pyrolyse von Kohlenwasserstoffen, insbesondere von Methan, Herstellung von Acetylen, Reformierung von Kohlenwasserstoffen, insbesondere Methan, Pyrolyse von Schwefelwasserstoff, Pyrolyse von Ammoniak und Hydrovergasung von Kohlenstoff.

In dem ersten Reaktor kann Umgebungsdruck, erhöhter Druck oder ein Unterdruck vorliegen. Die Reaktionstemperatur beträgt bevorzugt mehr als 2300°C. Die Reaktion ist vorrangig kinetisch bestimmt, so dass neben der Temperatur auch Parameter wie Druck oder Verweilzeit relevant sind. Die Temperatur im ersten Reaktor, insbesondere im Plasma, liegt bevorzugt in einem Bereich von 2000°C bis 5000°C. Der Druck im ersten Reaktor, im Verbindungsstück und/oder im zweiten Reaktor beträgt bevorzugt 1 Pa bis 1 MPa absolut, mehr bevorzugt 0,005 MPa bis 0,2 MPa absolut, also 50 mbar bis 2 bar absolut. Die Verweilzeit wird insbesondere durch die Dimensionierung des zweiten Reaktors bestimmt und bevorzugt an die Reaktionskinetik angepasst. Die Verweilzeit im ersten Reaktor und/oder im zweiten Reaktor beträgt bevorzugt jeweils weniger als 1 Sekunde. Weiter bevorzugt liegt die Verweilzeit im ersten Reaktor und/oder im zweiten Reaktor jeweils in einem Bereich von 10 Millisekunden bis 500 Millisekunden.

Die chemische Reaktion kann die plasmaindizierte Pyrolyse von Kohlenwasserstoffen, insbesondere Methan sein. In einer weiteren Ausführungsform kann eine Methanreformierung unter Einsatz von Kohlenstoffdioxid und/oder Wasserdampf erfolgen. Weiterhin kann die chemische Reaktion die Pyrolyse von Schwefelwasserstoff oder Ammoniak sein.

Bevorzugt werden das erste Eingangsgas an dem mindestens einen Einlass des ersten Abschnitts und das zweite Eingangsgas an dem mindestens einen Einlass des zweiten Abschnitts dem ersten Reaktor zugeführt und der Produktstrom wird bevorzugt an dem Auslass dem zweiten Reaktor entnommen, wobei der erste Abschnitt auf einer dem Verbindungsstück gegenüberliegenden Seite des Fensters angeordnet ist. Das erste Eingangsgas und das zweite Eingangsgas werden bevorzugt jeweils tangential in den ersten Reaktor geführt und in dem ersten Reaktor gemischt und bilden unter Einwirkung der elektromagnetischen Wellen ein Plasma. Die Zufuhr, das Mischen und die Plasma-Bildung erfolgen bevorzugt in der angegebenen Reihenfolge. Insbesondere werden das erste Eingangsgas und das zweite Eingangsgas zunächst gemischt und dann den elektromagnetischen Wellen in Mischung ausgesetzt.

Das erste Eingangsgas enthält bevorzugt Kohlenwasserstoffe, insbesondere Methan, und/oder andere Reaktanten. Zusätzlich kann das erste Eingangsgas weitere Gase wie Wasserstoff, Inertgase und/oder Wasserdampf enthalten. Weiterhin kann das erste Eingangsgas einen Feststoff wie Kohlenstoff enthalten. Die Reaktanten werden mit dem ersten Eingangsgas bevorzugt in reiner Form, insbesondere in einer Konzentration von mehr als 99 Vol.-% zugegeben . Das erste Eingangsgas kann zu mehr als 99 Vol.-% aus Reaktanten, insbesondere Kohlenwasserstoffen, bestehen, zum Beispiel Methan. Das erste Eingangsgas umfasst bevorzugt Biogas, insbesondere ein Gemisch enthaltend Methan und Kohlenstoffdioxid, Abgas wie Fackelgas, insbesondere aus Raffinerien, und/oder ein sonstiges Gemisch von Kohlenwasserstoffen. Die Zugabe von Gemischen von Reaktanten ist verfahrensbedingt auch möglich. Das erste Eingangsgas kann beispielsweise 50 bis 60 Vol.-% Methan und 40 bis 50 Vol.-% Kohlenstoffdioxid umfassen, insbesondere daraus bestehen, bezogen auf das gesamte erste Eingangsgas, insbesondere, wenn Biogas eingesetzt wird.

Weiterhin kann das erste Eingangsgas beispielsweise 3 bis 95 Vol.-% Kohlenwasserstoffe und 5 bis 97 Vol.-% Wasserstoff umfassen, insbesondere daraus bestehen, bezogen auf das gesamte erste Eingangsgas, insbesondere, wenn Abgas eingesetzt wird. Das erste Eingangsgas umfasst hier typischerweise 40 Vol.-% C₁₋₂-Kohlenwasserstoffe, die ein oder zwei Kohlenstoffatome aufweisen, 9 Vol.-% C₃₊-Kohlenwasserstoffe, die drei oder mehr Kohlenstoffatome aufweisen, 3 Vol.-% Kohlenstoffdioxid, 1 Vol.-% Stickstoff und 47 Vol.-% Wasserstoff.

Das erste Eingangsgas enthält weiter bevorzugt Methan und gegebenenfalls Wasserstoff und/oder Kohlenstoffdioxid. Das erste Eingangsgas weist bevorzugt eine Temperatur von nicht mehr als 600°C auf, insbesondere im Fall von Kohlenwasserstoffen wie Methan, um Rußbildung zu vermeiden. Die Temperatur des ersten Eingangsgases liegt bevorzugt in einem Bereich von 20°C bis 600°C, mehr bevorzugt in einem Bereich von 300°C bis 1500°C.

Das zweite Eingangsgas enthält bevorzugt ein Inertgas, Wasserdampf und/oder Wasserstoff und gegebenenfalls Kohlenwasserstoffe, insbesondere Methan, oder ein Gemisch verschiedener Reaktanten, insbesondere verschiedener Kohlenwasserstoffe. Die Kohlenwasserstoffe liegen in dem zweiten Eingangsgas insbesondere in nur kleiner Menge beziehungsweise geringer Konzentration vor. Insbesondere ist die Konzentration an Kohlenwasserstoffen im zweiten Eingangsgas kleiner als im ersten Eingangsgas. Entsprechend wird das erste Eingangsgas bevorzugt im ersten Reaktor durch das zweite Eingangsgas verdünnt.

Die Konzentration an Kohlenwasserstoffen, insbesondere einschließlich Methan, beträgt in der Summe der Eingangsgase, also des ersten Eingangsgases und des zweiten Eingangsgases, bevorzugt 50 Vol.-% oder weniger. Dadurch wird die in dieser Phase des Prozesses unerwünschte Bildung von festen Reaktionsprodukten wie Kohlenstoff, die die Ausbildung des Plasmas behindern würde, vermieden.

Das Volumenverhältnis des ersten Eingangsgases zum zweiten Eingangsgas, insbesondere des ersten Eingangsgasstroms zum zweiten Eingangsgasstrom, ist bevorzugt kleiner als 1. Durch das Volumenverhältnis der beiden Eingangsströme wird die Position der Mischzone beeinflusst und eine Lage der Mischzone am Fenster, das für die elektromagnetischen Wellen durchlässig ist, vermieden. Eine Zugabe des ersten Eingangsgases und des zweiten Eingangsgases in einem Verhältnis von 1, wobei das erste Eingangsgas reinen Reaktanten, zum Beispiel reines Methan enthält, resultiert zum Beispiel in einer Mischung enthaltend 50 Vol.- % an dem Reaktanten beziehungsweise Methan in dem ersten Reaktor. In einem weiteren Beispiel kann das zweite Eingangsgas zur Anreicherung mit Kohlenstoffdioxid eingesetzt werden um ein stöchiometrischen Verhältnis von 1:1 und eine Konzentration von 50 Vol.-% Methan zu erzielen, wenn zum Beispiel Biogas zu Synthesegas umgesetzt wird.

Das Inertgas enthält oder besteht aus bevorzugt Stickstoff und/oder Argon.

Der Produktstrom enthält bevorzugt den Feststoff, insbesondere Kohlenstoff. Der Produktstrom kann zumindest teilweise zurückgeführt werden, insbesondere in den ersten Reaktor. Dadurch kann die Ausbeute gesteigert werden. Bei der Rückführung kann ein gegebenenfalls im Produktstrom vorhandener Feststoff von der Gasphase abgetrennt werden. Beispielsweise kann bei der Methanpyrolyse pulverförmiger Kohlenstoff bei der Rückführung abgetrennt werden. Dadurch wird einer Behinderung der Plasmabildung entgegengewirkt. Bei der Hydrovergasung von Kohlenstoff wird zum Beispiel bevorzugt auf eine Abtrennung von Feststoff bei der Rückführung verzichtet. Bei der Hydrovergasung stellt der Kohlenstoff einen Reaktanten dar, der der Umsetzung nicht zu entziehen ist. Zur Abtrennung des Feststoffs können Filter und/oder Zyklone eingesetzt werden. Die Rückführung des Produktstroms kann als Teil des ersten Eingangsgases und/oder des zweiten Eingangsgases erfolgen. Die Wahl hängt von der im Reaktor durchgeführten Reaktion ab. Im Fall einer Hydrovergasung von Kohlenstoff wird die Rückführung des Produktstroms zum Beispiel bevorzugt über das erste Eingangsgas realisiert, um die Plasmabildung zu optimieren.

In einer bevorzugten Ausführungsform wird Wärme von dem Produktstrom auf das erste Eingangsgas und/oder das zweite Eingangsgas übertragen. Dazu wird insbesondere der Produktstrom einem ersten Raum eines Wärmetauschers zugeführt und das erste Eingangsgas, insbesondere ein erster Eingangsgasstrom, und/oder das zweite Eingangsgas, insbesondere ein zweiter Eingangsgasstrom, werden einem zweiten Raum des Wärmetauschers zugeführt, so dass ein Wärmeübertrag von dem Produktstrom auf das erste Eingangsgas und/oder das zweite Eingangsgas stattfindet. Dadurch wird eine Wärmerückgewinnung realisiert, das Energiemanagement bezüglich des Reaktors verbessert und die Effizienz des Verfahrens erhöht. Der für das Plasma benötigte Stromverbrauch wird reduziert. Bevorzugt ist der Wärmeübertrag einer möglichen Feststoffabtrennung vorgeschaltet.

Das dritte Eingangsgas enthält bevorzugt Wasserstoff und/oder Stickstoff.

Das Verfahren kann zur Entfernung und Immobilisierung von Kohlenstoffdioxid (CO₂) und/oder einem Abgas aus der Atmosphäre eingesetzt werden und zusätzlich folgende Schritte umfassen:
a) Umwandeln des Kohlenstoffdioxids aus der Atmosphäre und/oder einem Abgas in Biomasse mittels Photosynthese, bevorzugt auf Agrarflächen, insbesondere in einem Gewächshaus,
b) Durchführen einer Biogasreaktion, bei der die in Schritt a) erzeugte Biomasse zu Biogas, enthaltend Methan und Kohlenstoffdioxid, umgesetzt wird, insbesondere in einem Biogasreaktor,
c) Abtrennen des Methans aus dem erhaltenen Biogas,
d) Spalten des Methans in Kohlenstoff und Wasserstoff, wobei der Kohlenstoff als Feststoff gewonnen wird,
e) Auffangen des erhaltenen Kohlenstoffs und
f) Deponieren des erhaltenen Kohlenstoffs.

Die Schritte a) bis f) umfassen drei biologische beziehungsweise chemische Reaktionen, um CO₂ aus der Luft zu entfernen. Dies sind die Photosynthese in Schritt a), die Biogasreaktion, insbesondere eine anaerobe Vergärung in Schritt b) und das Spalten des Methans, insbesondere eine Pyrolyse von Methan in Schritt d).

Schritt d) wird bevorzugt in der erfindungsgemäßen Vorrichtung durchgeführt.

Schritt a) zur Herstellung der Biomasse kann in einem Agrarprozess, also auf einer landwirtschaftlich betriebenen Fläche, und/oder in einem Gewächshaus stattfinden.

Als Biomasse werden insbesondere Substanzen und deren Gemische definiert, die mit der Photosynthese erzeugt werden. Diese werden auch als primäre Biomasse bezeichnet. Unter der Biomasse werden weiterhin auch Stoffe und deren Gemische verstanden, die in Folge der Nutzung der primären Biomasse entstanden sind und biogenen Charakter beibehalten haben. Die Biomasse kann insbesondere Stroh, Forstreste, Wirtschaftsdünger, Nahrungsmittelreste und/oder kommunale Abfälle enthalten.

Bei dem Abgas, dessen CO₂ in Schritt a) gegebenenfalls in Biomasse umgewandelt wird, kann es sich beispielsweise um ein Abgas handeln, das beim Verbrennen von fossilen Brennstoffen in einem Kraftwerk entsteht. Das Abgas kann beispielsweise auch ein Nebenprodukt sein, welches in einem industriellen Herstellungsprozess entsteht, oder das Abgas kann ein Fördergas sein, welches bei der Förderung fossiler Energieträger wie Kohle, Erdöl oder Erdgas anfällt. Das Abgas, das Schritt a) zugeführt wird, ist insbesondere ein Gemisch, das CO₂ umfasst. Des Weiteren kann das Abgas als weitere Komponenten mindestens ein Inertgas wie Stickstoff oder Argon umfassen und gegebenenfalls Wasserdampf.

Bevorzugt entstammt das in Schritt a) eingesetzte Abgas zumindest teilweise der Verwendung des in Schritt d) erzeugten Wasserstoffs beziehungsweise des in Schritt b) gebildeten Biogases, insbesondere des Methans. Gegebenenfalls wird das Abgas in einer Vorbehandlung entschwefelt, von den anderen Verunreinigungen gereinigt und/oder entstaubt. Weiter bevorzugt besteht das in Schritt a) eingesetzte Abgas ausschließlich aus Abgas, das innerhalb des Verfahrens zurückgeführt wird. Mehr bevorzugt entstammt das in Schritt a) eingesetzte Abgas ausschließlich dem in Schritt b) erzeugten Biogas, insbesondere der Verwendung des in Schritt d) erzeugten Wasserstoffs. Entsprechend kann das Abgas dem Biogas direkt oder indirekt, also nach anschließender Spaltung des Methans, entstammen.

Die Biogasreaktion in Schritt b) umfasst insbesondere eine anaerobe Vergärungsreaktion. Bei der anaeroben Vergärung wird die Biomasse zu Biogas, also einem Gemisch aus überwiegend Methan und Kohlenstoffdioxid, umgewandelt. Das in Schritt b) erzeugte Biogas enthält bevorzugt in Summe mindestens 90 Vol.-%, weiter bevorzugt mindestens 95 Vol.-%, Methan und CO₂, bezogen auf das gesamte Biogas
Als Biomasse werden in Schritt b) bevorzugt Pflanzen, insbesondere C₃-Pflanzen, Bioabfälle, Wirtschaftsdünger, wie Mist und/Gülle, und/oder kommunale Abfälle eingesetzt. Die in Schritt a) erzeugte Biomasse kann direkt in Schritt b) eingesetzt werden. So können in Schritt a) erzeugte Pflanzen direkt der Biogasreaktion in Schritt b) zugeführt werden. Alternativ kann die in Schritt a) erzeugte Biomasse zumindest teilweise zuerst einer Benutzung oder Umwandlung ausgesetzt sein, so dass diese in Schritt b) als Bioabfälle, kommunale Abfälle oder Dünger vorliegt.

Die Biogasreaktion wird bevorzugt in einem geschlossenen Behälter ausgeführt, der auch als Fermenter bezeichnet wird. Vor Durchführung der Biogasreaktion kann die Biomasse zerkleinert und gegebenenfalls sortiert werden. Die Biomasse kann dem Fermenter kontinuierlich zugeführt werden. Die Verweilzeit der Biomasse in dem Fermenter beträgt bevorzugt mehr als einen Tag. Das produzierte Biogas sammelt sich insbesondere in einem oberen Bereich des Fermenters über einer Flüssig- und Feststoffphase an. Das Biogas enthält bevorzugt mindestens 40 Vol.-%, weiter bevorzugt 50 Vol.-% bis 75 Vol.-%, insbesondere 62 Vol.-% bis 75 Vol.-% Methan, bezogen auf das gesamte Biogas. Weiterhin enthält das Biogas CO₂ und gegebenenfalls Wasserdampf, Schwefelwasserstoff und/oder Ammoniak.

Das erzeugte Biogas enthält CO₂, das in einer Aufbereitung des Biogases, insbesondere einer CO₂-Abtrennvorrichtung, abgetrennt und der Photosynthese erneut zugeführt werden kann. Schritt c) kann entsprechend auch als Aufarbeitung des Biogases bezeichnet werden. Zur Abtrennung des Kohlenstoffdioxids können zum Beispiel Polyimid-Hohlfaser-Membranen verwendet werden. Bevorzugt wird das CO₂ aus dem erhaltenen Biogas nach der Abtrennung von Methan gemäß Schritt c) in Schritt a) zurückgeführt. Ferner kann das CO₂ aus dem erhaltenen Biogas nach der Abtrennung von Methan, gemäß Schritt c), verflüssigt werden und/oder das CO₂ aus dem erhaltenen Biogas kann nach der Abtrennung von Methan, gemäß Schritt c), zu einem Kohlenwasserstoffgemisch umgesetzt werden, insbesondere einer Fischer-Tropsch-Vorrichtung zugeführt werden.

Bevorzugt wird in einem Schritt g) CO₂ aus dem Biogas, insbesondere nach der Abtrennung von Methan gemäß Schritt c), mit in Schritt d) erzeugtem Wasserstoff zu einem Kohlenwasserstoffgemisch umgesetzt, wobei das Kohlenwasserstoffgemisch insbesondere Kerosin, Benzin und/oder Wachse enthält.

Bevorzugt erfolgt das Abtrennen des Methans in Schritt c) physikalisch, insbesondere mittels Kondensation, Adsorption und/oder einem Membranverfahren. Das Abtrennen des Methans aus dem erhaltenen Biogas in Schritt c) wird bevorzugt kontinuierlich durchgeführt.

Für das Abtrennen des Methans wird insbesondere Druckwechseladsorption, Temperatur-Vakuum-Adsorption, chemische Adsorption, Membrantrennung, Druckgaswäscheverfahren und/oder Wechselkonzentration-Adsorption eingesetzt.

Nach dem Abtrennen in Schritt c) enthält der Stoffstrom des abgetrennten Methans bevorzugt mehr als 95 Vol.-% Methan und weiter bevorzugt nicht mehr als 1 Vol.-% CO₂.

Die zum Betrieb der erfindungsgemäßen Vorrichtung benötigte Energie wird bevorzugt in Form von Strom aus erneuerbaren Quellen wie Wind, Biogas und/oder Photovoltaik bereitgestellt, der insbesondere derzeit im Stromnetz nicht abgenommen werden kann. Alternativ kann ein Teil des Biogases beziehungsweise des gewonnenen Wasserstoffs für das Bereitstellen der für die Spaltung notwendigen Energie verwendet werden.

Bevorzugt ist Schritt d) ein Filter nachgeschaltet, in dem der Kohlenstoff vom Wasserstoff getrennt wird. Die Umsetzung des Methans in Wasserstoff und Kohlenstoff erfolgt bevorzugt mit einer Ausbeute von ca. 96 bis 97 %.

Bevorzugt wird zumindest ein Teil des in Schritt d) erzeugten Wasserstoffs bzw. des in Schritt b) erzeugten Biogases, als Energiequelle für das Spalten des Methans benutzt werden.

Bevorzugt wird der in Schritt d) erzeugte Wasserstoff zumindest teilweise als Ausgangsstoff für Synthesen in der chemischen Industrie, als Energieträger für die Erzeugung von elektrischem Strom, Wärme, gegebenenfalls Kälte und/oder als Kraftstoff für Fahrzeuge verwendet.

Der Kohlenstoff wird bevorzugt gesammelt, zu einer Deponiestätte transportiert und dauerhaft eingelagert beziehungsweise endgelagert. Bevorzugt wird der in Schritt d) erhaltene Kohlenstoff vollständig deponiert.

Weiter bevorzugt wird der in Schritt d) erhaltene Kohlenstoff vor dem Deponieren in Schritt f) mit anderen Komponenten, insbesondere weiteren Feststoffen, so vermengt, dass eine energetische Nutzung nicht mehr möglich ist, um den Kohlenstoff endgültig zu immobilisieren und besonders sicher verwahren zu können. Als weitere Feststoffe können beispielsweise Sand, Lehm, Kies, Bauschutt, Schlacken, Steine, Abfälle, insbesondere aus Industriedemontagen, oder eine Kombination mehrerer dieser Materialien eingesetzt werden. Entsprechend wird der in Schritt d) erhaltene Kohlenstoff bevorzugt immobilisiert und durch das Vermischen des erhaltenen Kohlenstoffs mit einem weiteren Feststoff wird eine dauerhafte Immobilisierung sichergestellt. Das gebildete Feststoffgemisch, das den erhaltenen Kohlenstoff mit mindestens einem weiteren Feststoff umfasst, wird insbesondere geologisch und dauerhaft in einer Kohlenstoffsenke, wie zum Beispiel einem Bergwerk, gelagert.

Das Deponieren in Schritt f) erstreckt sich bevorzugt auf mindestens 30 Jahre, weiter bevorzugt mindestens 50 Jahre.

Ferner kann das Verfahren beziehungsweise die Vorrichtung zur Speicherung und zum Transport von elektrischer Energie eingesetzt werden. In einer bevorzugten Ausführungsform wird die Vorrichtung in einem Speicher- und/oder Transportverfahren eingesetzt, das die folgenden Schritte umfasst, insbesondere umfasst das erfindungsgemäße Verfahren zusätzlich die folgenden Schritte:
i. Erzeugung von Methan aus Wasser und Kohlenstoff unter Verwendung von elektrischer Energie,
ii. Speichern des Methans,
iii. Spalten des Methans in Wasserstoff und Kohlenstoff, wobei der Wasserstoff zur Energieerzeugung und/oder als Reaktant in einer chemischen Reaktionen verwendet wird oder
   Energieerzeugung durch Umsetzen des Methans in Kohlenstoff und Wasser in einer zyklischen Bromierungs-Oxidations-Prozess,
wobei der bei der Methanspaltung oder dem zyklischen Bromierungs-Oxidations-Prozess gemäß Schritt iii. anfallende Kohlenstoff aufgefangen wird und beim erneuten Durchlaufen des Verfahrens für die Methanerzeugung in Schritt i. verwendet wird, so dass ein geschlossener Kohlenstoffkreislauf entsteht.

Insbesondere wird die Erzeugung von Methan in Schritt i. und/oder das Spalten des Methans in Schritt iii. in der erfindungsgemäßen Vorrichtung ausgeführt.

Bevorzugt dient der Kohlenstoff im Speicher- und/oder Transportverfahren als Träger für Wasserstoff. Der Kohlenstoff wird hier insbesondere nicht als Brennstoff zur Energieerzeugung eingesetzt. Als Energieerzeugung wird in diesem Zusammenhang insbesondere die Stromerzeugung und/oder die Verwendung des durch die Spaltung des Methans gemäß Schritt iii. gewonnenen Wasserstoffs für weitere energetische Anwendungen, wie Heizung, Kühlung oder Antrieb von Fahrzeugen wie Autos, LKW, Bahnen oder Schiffen, verstanden.

In Schritt i. wird bevorzugt zunächst Synthesegas erzeugt, indem der, insbesondere pulverförmiger, Kohlenstoff mit Wasserdampf zu einem Gemisch enthaltend Kohlenstoffmonoxid und Wasserstoff umgesetzt wird. Die dazu erforderliche Energie wird bevorzugt mittels erneuerbaren Quellen erzeugt. Das Synthesegas wird anschließend bevorzugt mit Wasserstoff, der insbesondere mittels Elektrolyse aus Wasser und Strom und erneuerbaren Quellen hergestellt wird, katalytisch zu Methan umgesetzt.

### Vorteile der Erfindung

Mit der erfindungsgemäßen Vorrichtung beziehungsweise dem erfindungsgemäßen Verfahren können das erste Eingangsgas und das zweite Eingangsgas, die das Reaktionsgemisch bilden, unabhängig voneinander in den ersten Reaktor eingeführt werden. Durch die bezüglich dem Fenster gegenüberliegende Zuführung des zweiten Eingangsgases wird das Reaktionssystem stabilisiert.

Die getrennte Zuführung von dem ersten Eingangsgas und dem zweiten Eingangsgas hat den Vorteil, dass die Reaktion erst nach dem Vermischen der beiden Ströme miteinander stattfindet und dies gezielt in einer Mischzone geschieht, in der die beiden wirbelförmigen Strömungen des ersten Eingangsgases beziehungsweise des zweiten Eingangsgases aufeinanderstoßen, intensiv vermischt werden und eine innere, wirbelartige Strömung bilden. Dadurch wird sichergestellt, dass ein homogenes Gemisch der Reaktanten in Plasma übergeht und keine Bypass-Effekte auftreten.

Das Plasma und bei der chemischen Reaktion gebildete Produkte werden in einem mittleren Bereich des ersten Reaktors stabilisiert, also mit einem Abstand von der Reaktorwand. Dies ist insbesondere von Vorteil, wenn die Reaktionsprodukte zumindest teilweise in fester Form vorliegen und dazu tendieren, sich an der Innenwand des Reaktors niederzuschlagen, wodurch es zu einer lokalen Überhitzung der Reaktorwand und zur Störung der Reaktorfunktionen kommen kann. Die Ausbildung der Wirbelströmung in der Nähe der Reaktorwand verhindert diese negativen Effekte.

**In** dem Verbindungsstück wird die chemische Reaktion annähernd adiabatisch fortgesetzt und es wird sichergestellt, dass das Plasma den zweiten Reaktor erreicht.

Durch den zweiten Reaktor wird die Verweilzeit im Kaskadensystem verlängert, um die chemische Reaktion abzuschließen. In dem zweiten Reaktor liegt bevorzugt kein elektromagnetisches Feld mehr vor. Über die Länge des ersten Reaktors und des zweiten Reaktors kann die optimale Verweilzeit eingestellt werden und erlaubt die Erreichung der gewünschten Reaktionsergebnisse.

Durch die wirbelförmigen Strömungen des Reaktionsgemischs werden Strömungsverhältnisse eingestellt, die denen eines idealen Rohrreaktors ähnlich sind. Somit kann die Reaktion adiabatisch durchgeführt werden, da eine axiale Vermischung innerhalb des Reaktors minimiert wird.

Die, insbesondere koaxiale, Anordnung des Innenrohrs im zweiten Reaktor wirkt der Ablagerung von festen Reaktionsprodukten an den Wänden des zweiten Reaktors entgegen, da die Fließgeschwindigkeit im äußeren Gasraum, also zwischen Innenrohr und Innenwand des zweiten Reaktors erhöht wird.

Anhand der nachstehenden Zeichnungen (Figuren 1 bis 5), der Bezugszeichenliste und der Patentansprüche sowie der Beispiele wird die Erfindung eingehender beschrieben.

Es zeigen:
- Figur 1: eine erste Ausführungsform der erfindungsgemäßen Vorrichtung,
- Figur 1a: eine Querschnittsansicht der ersten Ausführungsform der Vorrichtung,
- Figur 2: eine zweite Ausführungsform der erfindungsgemäßen Vorrichtung,
- Figur 2a: eine Querschnittsansicht der zweiten Ausführungsform der erfindungsgemäßen Vorrichtung,
- Figur 3: eine dritte Ausführungsform der erfindungsgemäßen Vorrichtung,
- Figur 3a: eine Querschnittsansicht der dritten Ausführungsform der erfindungsgemäßen Vorrichtung,
- Figur 4: eine vierte Ausführungsform der erfindungsgemäßen Vorrichtung,
- Figur 4a: eine Querschnittsansicht der vierten Ausführungsform der erfindungsgemäßen Vorrichtung,
- Figur 5: ein Schema der optionalen Wärmerückgewinnung und Feststoffabtrennung,
- Figur 6: ein Schema eines Verfahrens, in dem die erfindungsgemäße Vorrichtung 4 eingesetzt wird und
- Figur 7: ein Schema eines weiteren Verfahrens, in dem die erfindungsgemäße Vorrichtung 4 eingesetzt wird.

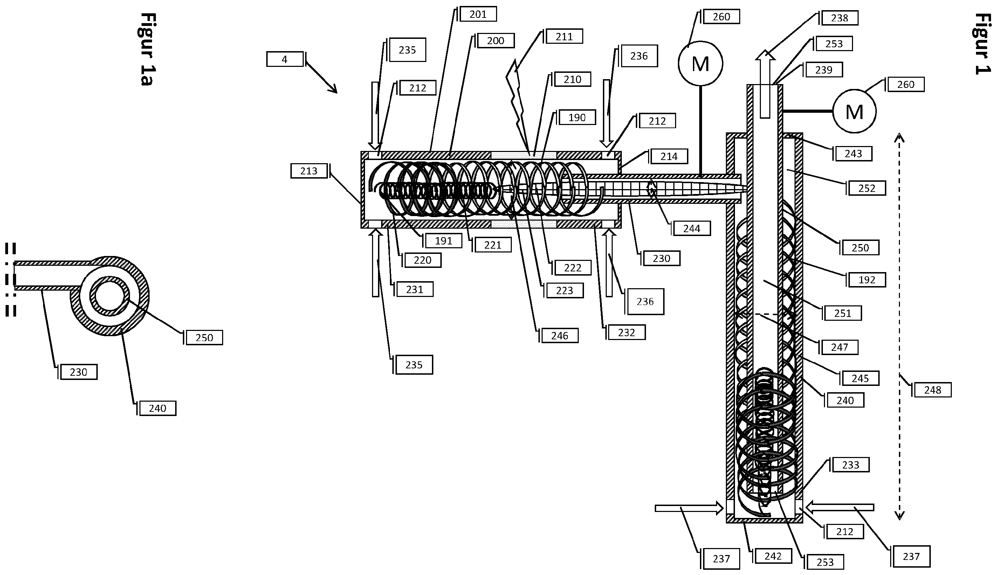

Figur 1 zeigt eine erste Ausführungsform der erfindungsgemäßen Vorrichtung 4 im Längsschnitt. Die Vorrichtung 4 weist einen ersten Reaktor 200 und einen zweiten Reaktor 240 auf, die durch ein Verbindungsstück 230 fluidisch miteinander verbunden sind.

Der erste Reaktor 200 ist durch eine erste Mantelfläche 201 und erste Endflächen 213, 214 begrenzt. Durch gegenüberliegende Einlässe 212 an einem ersten Abschnitt 231 der ersten Mantelfläche 201 wird ein erstes Eingangsgas 235 tangential in den ersten Reaktor 200 zugeführt, wobei sich eine erste Wirbelströmung 220 ausbildet. Die erste Wirbelströmung 220 trifft auf eine entgegengesetzt strömende zweite Wirbelströmung 190 eines zweiten Eingangsgases 236, das an zwei Einlässen 212 an einem zweiten Abschnitt 232 der ersten Mantelfläche 201 ebenfalls tangential in den ersten Reaktor 200 zugeführt wird.

Die erste Wirbelströmung 220 und die zweite Wirbelströmung 190 treffen in einer Mischzone 221 in dem ersten Reaktor 200 aufeinander, wo das erste Eingangsgas 235 und das zweite Eingangsgas 236 miteinander vermischt werden und eine dritte Wirbelströmung 191 bilden, mit der das entstandene Eingangsgasgemisch an einem Fenster 210 des ersten Reaktors 200 elektromagnetischen Wellen 211 ausgesetzt wird, so dass ein Plasma 223 entsteht, das den ersten Reaktor 200 über das Verbindungsstück 230 verlässt.

Das Plasma 223 wird in einer Plasmazone 222 am Fenster 210 indiziert und dabei durch die zweite Wirbelströmung 190 gestützt, die das Plasma 223 in Wandnähe des ersten Reaktors 200 umgibt. Dann wird das Plasma 223 tangential in den zweiten Reaktor 240 eingeführt. Der zweite Reaktor 240 weist ein Innenrohr 250 auf, so dass ein innerer Gasraum 251 und ein äußerer Gasraum 252 gebildet werden. Das Plasma 223 tritt in den äußeren Gasraum 252 ein und bildet eine vierte Wirbelströmung 192 um das Innenrohr 250 herum.

Der zweite Reaktor 240 wird durch eine zweite Mantelfläche 245 und zweite Endflächen 242, 243 begrenzt. An einer von dem Verbindungsstück 230 entfernten der zweiten Endflächen 242 sind an einem dritten Abschnitt 233 Einlässe 212 vorgesehen, durch die ein drittes Eingangsgas 237 zur Kühlung zugeführt wird.

Ein Produktstrom 238 verlässt den zweiten Reaktor 240 durch einen Auslass 239 an einem offenen Ende 253 des Innenrohrs 250.

Das Verbindungsstück 230 weist einen konstanten Durchmesser 244 auf. Der erste Reaktor 200 weist einen konstanten ersten Reaktordurchmesser 246 und der zweite Reaktor 240 einen konstanten zweiten Reaktordurchmesser 247 auf. Weiterhin besitzt der zweite Reaktor 240 eine Gesamtlänge 248.

Insbesondere durch die Ausbildung der ersten Wirbelströmung 220 und der zweiten Wirbelströmung 190, die außerhalb des Fensters 210 im ersten Reaktor 200 im Gegenstrom aufeinandertreffen, wird eine homogene Eingangsgasmischung erzielt, die anschließend zum Ablauf der chemischen Reaktion in das Plasma 223 an dem Fenster 210 überführt wird. Weiterhin werden durch die erste Wirbelströmung 220 und die zweite Wirbelströmung 190 Feststoffablagerungen an der ersten Mantelfläche 201 und insbesondere an dem Fenster 210 vermieden.

Das Innenrohr 250 in dem zweiten Reaktor 240 sorgt für eine erhöhte Strömungsgeschwindigkeit in dem äußeren Gasraum 252, so dass auch im zweiten Reaktor 240 Feststoffablagerungen vermieden werden. Feststoffablagerungen werden darüber hinaus durch Vibrationsvorrichtungen 260 an dem Verbindungsstück 230 und dem Innenrohr 250 unterbunden.

Figur 1a zeigt eine Querschnittsansicht des zweiten Reaktors 240 mit dem Innenrohr 250 und dem Verbindungsstück 230 gemäß der ersten Ausführungsform der Vorrichtung 4. In dieser Querschnittsansicht ist der Anschluss des Verbindungsstücks 230 in tangentialer Ausrichtung an den zweiten Reaktor 240 erkennbar.

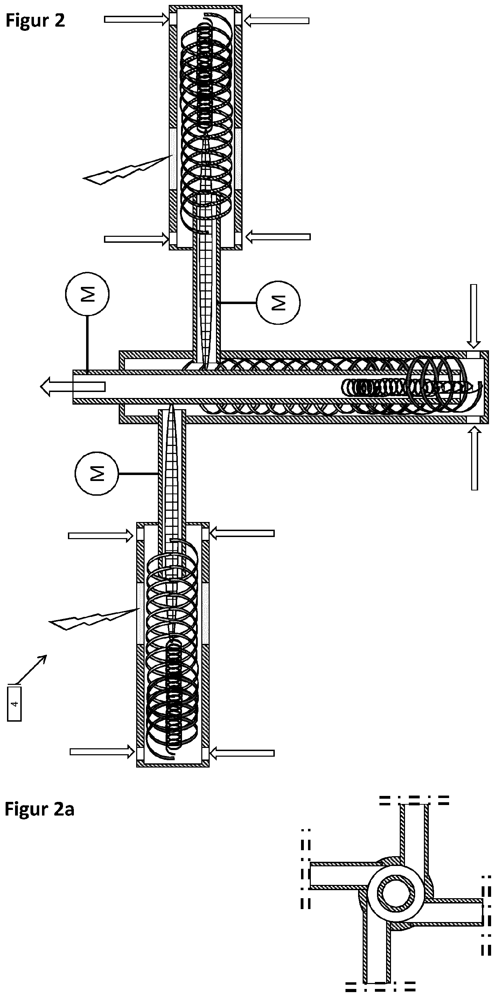

Figur 2 zeigt eine zweite Ausführungsform der erfindungsgemäßen Vorrichtung 4, die im Wesentlichen der ersten Ausführungsform gemäß Figur 1 entspricht, mit dem Unterschied, dass an den zweiten Reaktor 240 vier erste Reaktoren 200 mit jeweils einem Verbindungsstück 230 angeschlossen sind.

Figur 2a zeigt eine Querschnittsansicht des zweiten Reaktors 240 gemäß der zweiten Ausführungsform mit vier tangential angeordneten Verbindungsstücken 230.

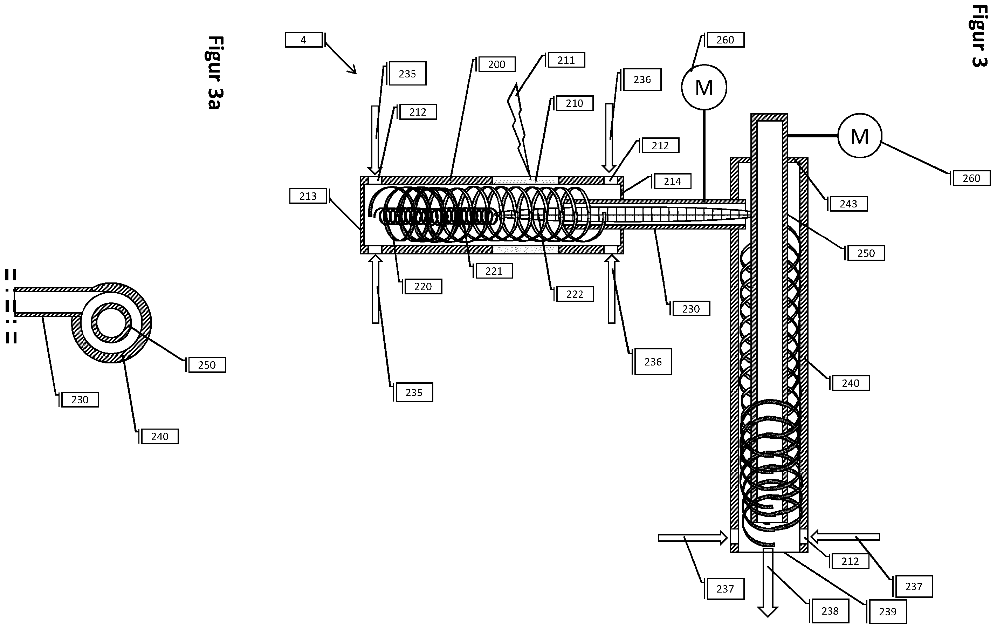

Figur 3 zeigt eine dritte Ausführungsform der erfindungsgemäßen Vorrichtung 4, die sich in der Ausführung des zweiten Reaktors 240 von der ersten Ausführungsform gemäß Figur 1 unterscheidet. Gemäß der zweiten Ausführungsform weist das Innenrohr 250 ein offenes Ende 253 und ein geschlossenes Ende 254 auf, wobei das offene Ende 253 an der von dem Verbindungsstück 230 entfernteren der zweiten Endfläche 242 angeordnet ist, an der sich in dieser Ausführungsform auch der Auslass 239 befindet, an dem der Produktgasstrom 238 entnommen wird. In der dritten Ausführungsform wird das Innenrohr 250 nicht von dem Produktstrom im inneren Gasraum 51 durchströmt. Es liegt lediglich die vierte Wirbelströmung 192 um das Innenrohr 250 herum vor.

Figur 3a zeigt eine Querschnittsansicht des zweiten Reaktors 240 mit dem Verbindungsstück 230 gemäß der dritten Ausführungsform.

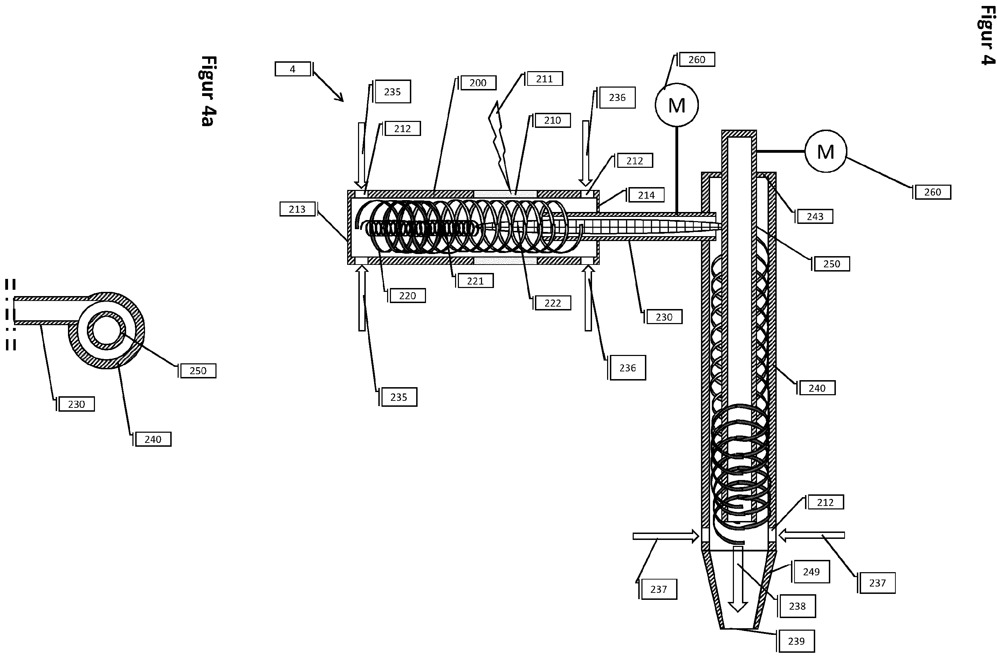

Figur 4 zeigt eine vierte Ausführungsform der erfindungsgemäßen Vorrichtung 4, die sich durch die Gestaltung des Auslasses 239 als ein konisches Ende 249 des zweiten Reaktors 240 von der dritten Ausführungsform gemäß Figur 3 unterscheidet.

Figur 4a zeigt eine Querschnittsansicht des zweiten Reaktors 240 mit dem Verbindungsstück 230 gemäß der vierten Ausführungsform.

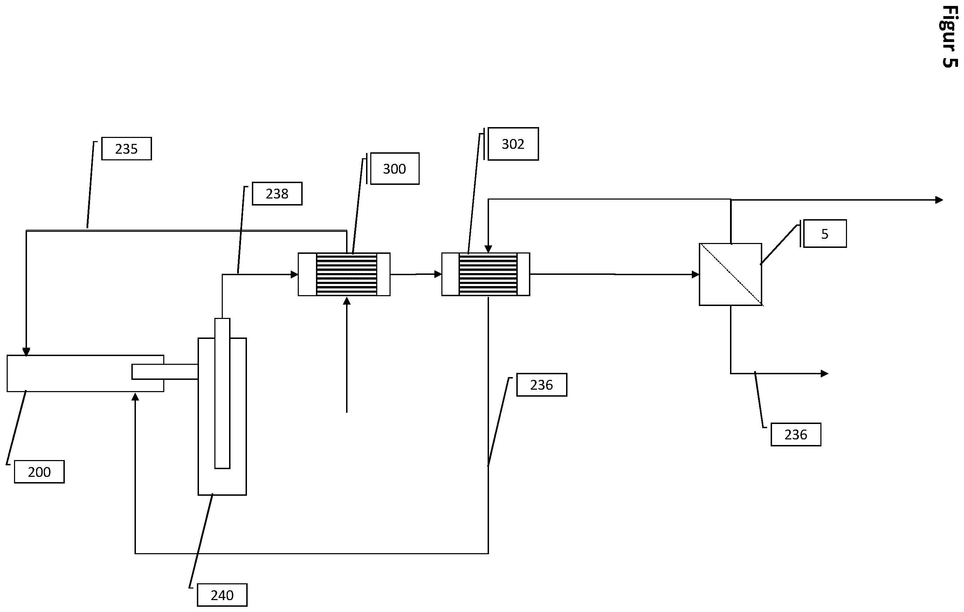

Figur 5 zeigt ein Schema der optionalen Wärmerückgewinnung und Feststoffabtrennung. Dem zweiten Reaktor 240 sind ein erster Wärmetauscher 300 und ein zweiter Wärmetauscher 302 nachgeschaltet, wobei der zweite Wärmetauscher 302 mit einem Filter 5 zur Feststoffabtrennung verbunden ist. Der Produktstrom 238, der dem zweiten Reaktor 240 entnommen wird, wird gekühlt. Dabei wird in dem ersten Wärmetauscher 300 zunächst Wärme an ein erstes Eingangsgas 235 abgegeben und in dem zweiten Wärmetauscher 302 wird Wärme von dem Produktstrom 238 auf ein zweites Eingangsgas 236 übertragen. Das erste Eingangsgas 235 wird von dem ersten Wärmetauscher 300 dem ersten Reaktor 100 zugeführt und das zweite Eingangsgas 236 von dem zweiten Wärmetauscher 302. Nach dem zweiten Wärmetauscher wird der Produktstrom 238 über den Filter 5 geführt, wo ein Feststoff, insbesondere Kohlenstoff 106, abgetrennt wird. Das verbleibende gasförmige Produktgas wird teilweise als zweites Eingangsgas 236 in den ersten Reaktor 200 zurückgeführt.

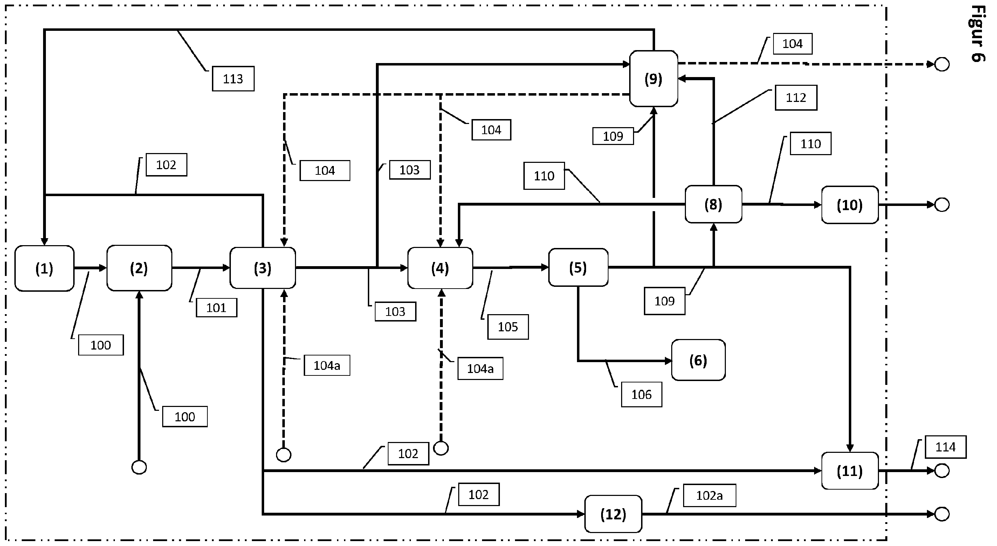

Figur 6 zeigt ein Schema eines Verfahrens, in dem die erfindungsgemäße Vorrichtung 4 als Reaktor eingesetzt wird.

In einem Schritt a) wird Biomasse 100 mittels Photosynthese aus CO₂ 102 erzeugt. Dies geschieht beispielsweise durch Pflanzenwachstum auf einem Feld oder alternativ oder zusätzlich durch den Anbau von Pflanzen in einem Gewächshaus 1.

Für die Steigerung des Pflanzenwachstums kann die Luft im Gewächshaus 1 mit CO₂ 102 angereichert und erwärmt werden. Das CO₂ 102 kann separat und/oder als Teil eines Abgases 113 dem Gewächshaus 1 zugeführt werden. Dabei wird das CO₂ 102 bevorzugt aus weiteren Schritten des Verfahrens rückgeführt, insbesondere aus einer Aufarbeitung 3 von im Verfahren hergestelltem Biogas 101 und als Teil des Abgases 113 aus einem Blockheizkraftwerk (BHKW) 9.

Die Biomasse 100 wird in einem Schritt b) des Verfahrens, insbesondere mit einer anaeroben bakteriellen Reaktion, zu Biogas 101, das Methan 103 und CO₂ 102 enthält, vergärt. Die Reaktion wird insbesondere kontinuierlich in einem Mischbehälter, der einen Biogasreaktor 2 darstellt, durchgeführt.

In einem Schritt c) des Verfahrens wird das Biogas 101 einer Aufarbeitung 3 zugeführt, wobei Methan 103 in Form eines Methan-reichen Stroms, von einem CO₂ 102 enthaltenden Strom abgetrennt wird. Das CO₂ 102, zum Beispiel der gesamte CO₂ 102 enthaltende Strom, kann in das Gewächshaus 1 zurückgeführt werden, um die Luft im Gewächshaus 1 mit CO₂ 102 anzureichern. Alternativ oder ergänzend zur Rückführung des CO₂ 102 von der Aufarbeitung 3 in das Gewächshaus 1 kann das CO₂ 102 zu einer Fischer-Tropsch-Vorrichtung 11 geleitet werden.

In einem Schritt d) des Verfahrens wird das Methan 103 von der Aufarbeitung 3 der erfindungsgemäßen Vorrichtung 4 zugeführt. In der Vorrichtung 4 wird Methan 103 in seine Bestandteile Kohlenstoff als Feststoff 106 und Wasserstoff 110 gespalten.

Die Umsetzung des Methans 103 ist nicht vollständig und andere Kohlenwasserstoffe fallen in geringen Mengen ebenfalls an. Am Ausgang des Reaktors 4 entsteht ein Produktgasgemisch 105, das gasförmiges Methan 103, Wasserstoff 110 und andere Kohlenwasserstoffe sowie Kohlenstoff als Feststoff 106 enthält.

Zum Betrieb der Vorrichtung 4 wird bevorzugt Energie wie elektrischer Strom 104 aus erneuerbaren Quellen eingesetzt, insbesondere aus Windenergie und Photovoltaik. Der elektrische Strom 104 kann alternativ oder zusätzlich in der BHKW-Anlage 9 erzeugt werden.

Der Vorrichtung 4 ist ein Filter 5 nachgeschaltet, in dem die Trennung des Feststoffs 106 von einer Gasphase 109 erfolgt, die Wasserstoff 110 enthält.

Die Gasphase 109 kann gegebenenfalls zwischengelagert und als Brennbeziehungsweise Treibstoff der integrierten BHKW-Anlage 9 zugeführt werden. Weiterhin kann der in der BHKW-Anlage erzeugte elektrische Strom 104 netzstabilisierend ins öffentliche Stromnetz eingespeist werden.

Alternativ kann der Wasserstoff 110 in einer Wechseldruckadsorptionsanlage oder mittels Membranen 8 von in der Gasphase 109 vorhandenem Restgas 112 separiert werden. Ein Teil des Wasserstoffs 110 kann in den Reaktor 4 zurückgeführt werden, ein weiterer Teil als Produkt weiterverwertet werden.

Alternativ oder ergänzend kann die Gasphase 109 der Fischer-Tropsch-Vorrichtung 11 zugeführt werden. Hier kann die Gasphase 109 zusammen mit CO₂ 102, das der Aufarbeitung 3 entstammt, in ein Kohlenwasserstoffgemisch 114 umgewandelt werden. Das Kohlenwasserstoffgemisch 114 enthält bevorzugt Kerosin, Benzin, Wachse und Mischungen daraus.

Das nach der Abtrennung aus der Gasphase 109 verwendete und als Brennbeziehungsweise Treibstoff dienende Restgas 112 kann gegebenenfalls zwischengelagert und der BHKW-Anlage 9 zugeführt werden. Als Alternative zu einer externen Stromquelle kann der in der BHKW-Anlage 9 erzeugte elektrische Strom 104 für die Spaltung des Methans 103 in der Vorrichtung 4 genutzt werden. Weiterhin werden die in der BHKW-Anlage 9 erzeugten Abgase 113, insbesondere mit der dort erzeugten Wärme 115 bevorzugt dem Gewächshaus 1 zugeführt.

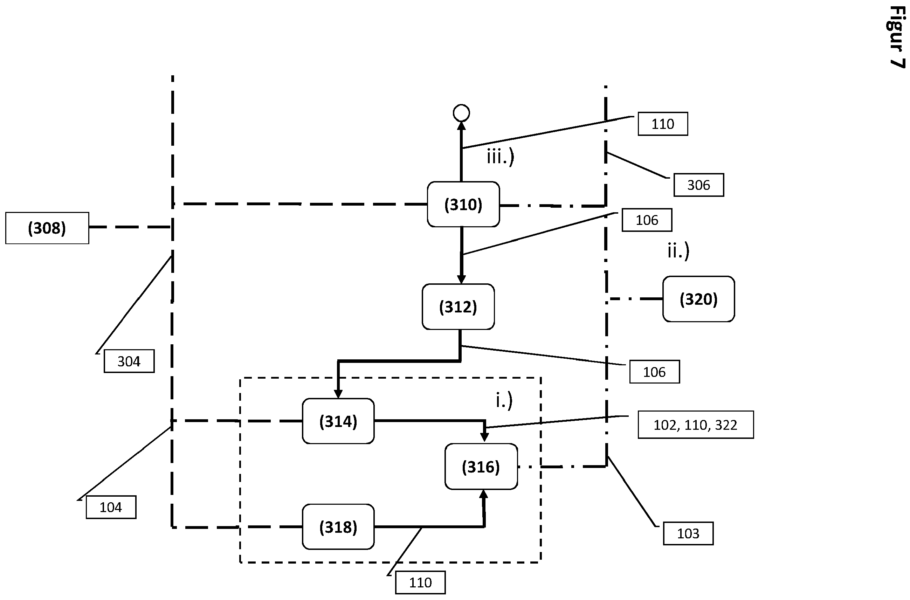

Figur 7 zeigt ein Schema eines weiteren Verfahrens, in dem die erfindungsgemäße Vorrichtung 4 eingesetzt wird. Schematisch dargestellt ist das Speicher- und/oder Transportverfahren, das ein Stromnetz 304 und ein Gasnetz 306 durch mehrere Verfahrensschritte miteinander verknüpft. Das Stromnetz 304 wir aus erneuerbaren Energien 308 wie zum Beispiel auf Basis von Windenergie oder Solarenergie gespeist. Elektrischer Strom 104 wird in einem Schritt i. in einer Hydrovergasung 314 von Kohlenstoff 106 und einer Elektrolyse 318 eingesetzt um Methan 103 zu erzeugen. Kohlenstoffdioxid 102, Kohlenstoffmonoxid 322 und Wasserstoff 110 werden dazu einer Methanisierung 316 zugeführt. Erzeugtes Methan wird gemäß einem Schritt ii. in einem Gasspeicher 320 gelagert und gemäß einem Schritt iii. in einem Wasserstoff-Generator 310 in Wasserstoff 110 und Kohlenstoff 106 gespalten. Der Kohlenstoff 106 wird in einem Kohlespeicher 312 zwischengelagert. Der Wasserstoff kann zur Erzeugung von Wärme, im Verkehr oder zur Stromerzeugung eingesetzt oder in der chemischen Industrie umgesetzt werden. Die erfindungsgemäße Vorrichtung 4 wird in der Hydrovergasung 314 und in dem Wasserstoff-Generator 310 eingesetzt.

### Beispiele

### Ausführungsbeispiel 1: Pyrolyse von Methan

Die Pyrolyse von Methan, das als Erdgas, synthetisches Erdgas (SNG) oder Biomethan eingesetzt werden kann, wird durch die nachstehende Reaktionsgleichung beschrieben:

CH₄ → C + 2 H₂

Es werden Methan und Wasserstoff, das hier auch als Plasmagas bezeichnet wird, eingesetzt. Ein Teil des Wasserstoffs wird am zweiten Abschnitt der Vorrichtung zugeführt, der restliche Wasserstoff wird zusammen mit Methan am ersten Abschnitt des ersten Reaktors, jeweils über zwei Einlässe zugeführt. Das Volumenverhältnis der Ströme am ersten Abschnitt beziehungsweise am zweiten Abschnitt liegt bei 1:1, so dass sich die Mischzone der beiden Ströme mittig im ersten Reaktor befindet.

Der am zweiten Abschnitt zugeführte Wasserstoff bildet eine dünne, wirbelförmige Schicht an der Reaktorwand und am Fenster, das für die elektromagnetischen Wellen durchlässig ist. Sie verhindert, dass sich bei der Reaktion entstehende Kohlenstoffpartikel an der Wand des ersten Reaktors niederschlagen. Ihre Ablagerung würde dazu führen, dass elektromagnetische Wellen absorbiert werden, das Fenster überhitzt und der erste Reaktor zerstört wird.

Die Reaktion findet zunächst in der Mischzone und anschließend im Plasma statt. Der entstehende Kohlenstoff als Feststoff verlässt den ersten Reaktor gemeinsam mit dem Produktgasstrom, also der Gasphase, durch das Verbindungsstück. Der Durchmesser des Verbindungsstücks ist so dimensioniert, dass die Fließgeschwindigkeit der Zwei-Phasen-Strömung mindestens 20 m/s beträgt.

Die Plasmazone und das Verbindungsstück bilden einen nahezu (quasi-) adiabatischen Rohrreaktor. Die mittels der elektromagnetischen Wellen eingebrachte Energie wird adsorbiert und mit der chemischen Reaktion verbraucht.

### Ausführungsbeispiel 2: Acetylen-Herstellung

Wenn im ersten Reaktor ein Unterdruck, insbesondere ein Vakuum in einem Bereich von 50 bis 100 mbar absolut, vorliegt, wird im Plasma die Bildung von Acetylen aus Methan gemäß folgender Reaktionsgleichung begünstigt:

2 CH₄ → C₂H₂ + 3 H₂

Methan wird am ersten Eingang des ersten Reaktors zugegeben, Wasserstoff am zweiten Eingang. Ausgehend vom zweiten Abschnitt bilden sich zwei wandnahe Wirbelströmungen aus Methan und Wasserstoff entsprechend aus, die zunächst in die Mitte des ersten Reaktors geführt werden. Es kommt zur Vermischung beider Ströme, die im Bereich des Fensters Plasma bilden. Nach Ausbildung des Plasmas liegt eine Temperatur im Bereich von 3000 bis 3500 °C vor.

Die Ionisierung und Fragmentierung der Eingangsgase bei reduziertem Druck führt zur Bildung von Acetylen. Die durch den Unterdruck zwar unterdrückte Reaktion gemäß dem Ausführungsbeispiel 1 findet in reduziertem Umfang ebenfalls statt, so dass Kohlenstoff als Feststoff in kleinen Mengen gebildet wird. Auch hier wird der Ablagerung des Feststoffs durch die wirbelförmige Gasführung entgegengewirkt.

Um einen Zerfall des gebildeten Acetylens bei nur langsam absinkender Temperatur zu vermeiden, wobei fester Kohlenstoff und Wasserstoff gebildet werden würde, wird das Produktgas gequencht, wobei ein kaltes drittes Eingangsgas am dritten Abschnitt in zweiten Reaktor zugeführt wird. Als drittes Eingangsgas wird Wasserstoff eingesetzt.

### Ausführungsbeispiel 3: Trockenreformierung von Biogas

Als erstes und zweites Eingangsgas wird Biogas eingesetzt. Die Konzentration von Methan im Biogas variiert stark und liegt zwischen 40 und 75 Vol.-%. Die Reaktion verläuft gemäß der stöchiometrischen Gleichung:

CO₂ + CH₄ → 2 CO + 2 H₂

Um das äquimolare Verhältnis zwischen den beiden Reaktanten Methan und Kohlenstoffdioxid zu ermöglichen, wird in Abhängigkeit von der Methankonzentration zusätzlich Methan am ersten oder Kohlenstoffdioxid am zweiten Eingang zugegeben. Beispielsweise bei einer Methan-Konzentration von 70 Vol.-% im Biogas besteht das erste Eingangsgas aus einem Volumenteil Biogas und das zweites Eingangsgas aus einem Teil Biogas und 0,8 Volumenteil CO₂.

Das Plasma wird in der Mitte des ersten Reaktors sowie im Verbindungsstück stabilisiert und im zweiten Reaktor wird die Reformierungsreaktion adiabatisch fortgesetzt.

Die Reformierungsreaktion läuft in zwei Schritten ab. Zunächst wird Methan im Plasma in seine Bestandteile Wasserstoff und atomaren Kohlenstoff beziehungsweise Ruß gespalten. In einem zweiten Schritt reagiert Kohlenstoff mit Kohlenstoffdioxid gemäß dem Boudouard-Prinzip zu Kohlenstoffmonoxid:

C + CO₂ → 2 CO

Durch die hohe Temperatur im Plasma wird eine Verschiebung des Reaktionsgleichgewichts von Kohlenstoff und Kohlenstoffdioxid zu Gunsten der Kohlenstoffmonoxid-Bildung begünstigt. Durch die wirbelförmigen Strömungen im ersten Reaktor und im zweiten Reaktor wird eine Feststoffablagerung an den Wänden vermieden, so dass dieser in der Gasphase verbleibt und mit dem Kohlendioxid reagiert.

Um die hier unerwünschte Nebenreaktion

CO₂ + H₂ → CO + H₂O

zu unterbinden, wird der Produktgasstrom gequencht, indem kaltes Wasserstoff-Gas, im dritten Abschnitt als drittes Eingangsgas in den zweiten Reaktor zugeführt wird.

### Ausführungsbeispiel 4: Methan-Dampfreformierung

Methan wird mit Wasserdampf zu Synthesegas im Plasma umgesetzt:

CH₄ + H₂O_{(g)} → CO + 3 H₂

Methan wird als erstes Eingangsgas im ersten Abschnitt über zwei Zulässe in den ersten Reaktor zugeführt. Wasserdampf wird als zweites Eingangsgas am zweiten Abschnitt über zwei Zulässe in den ersten Reaktor eingeführt. Der Wasserdampf bildet einen wirbelförmigen Strom in der Nähe der Wand des ersten Reaktors und stabilisiert das Plasma. Der Wasserdampf und das Methan werden beim Aufeinandertreffen der beiden Wirbel gemischt und bilden einen inneren Wirbel, der am Fenster ein Methan-Wasserdampf-Plasma bildet. Dort wird das Eingangsgasgemisch bei hohen Temperaturen ionisiert und zerfällt in kleinere Fragmente, also Radikale. Das Plasma wird über das Verbindungsstück in den zweiten Reaktor überführt und die Radikale bilden die Reaktionsprodukte, die als Produktstrom entnommen werden.

### Ausführungsbeispiel 5: Hydrovergasung von Kohlenstoff

Unter Anwendung von Plasma wird die Umsetzung von pulverförmigem Kohlenstoff mit Wasserdampf zu Synthesegas durchgeführt:

C + H₂O_{(g)} +H₂ → CO + 2 H₂

Die Reaktion ist endotherm. Um die Plasmabildung zu begünstigen wird Wasserstoff als Plasmagas zusätzlich verwendet. Als erstes Eingangsgas wird dem ersten Reaktor am ersten Abschnitt ein Gemisch aus Kohlenstoff und Wasserdampf zugeführt. Wasserdampf wird im Überschuss, verglichen mit stöchiometrischem Verhältnis der Reaktanten, verwendet. Wasserstoff wird als zweites Eingangsgas am zweiten Abschnitt zugeführt. Am ersten Abschnitt des ersten Reaktors wird außerdem der Kohlenstoff zugegeben. Dadurch wird gewährleistet, dass der pulverförmige Kohlenstoff die Plasmabildung nicht beeinträchtigt.

### Bezugszeichenliste

- 1: Gewächshaus
- 2: Biogasreaktor
- 3: Aufarbeitung
- 4: Vorrichtung
- 5: Filter
- 8: Membranen
- 9: Blockheizkraftwerk
- 11: Fischer-Tropsch-Vorrichtung
- 100: Biomasse
- 101: Biogas
- 102: CO₂
- 103: Methan
- 104: Elektrischer Strom
- 105: Produktgasgemisch
- 106: Feststoff (Kohlenstoff)
- 109: Gasphase
- 110: Wasserstoff
- 112: Restgas
- 113: Abgas
- 114: Kohlenwasserstoffgemisch
- 115: Wärme
- 190: Zweite Wirbelströmung
- 191: Dritte Wirbelströmung
- 192: Vierte Wirbelströmung
- 200: Erster Reaktor
- 201: Erste Mantelfläche
- 210: Fenster
- 211: Elektromagnetische Wellen
- 212: Einlass
- 213, 214: Erste Endflächen
- 220: Erste Wirbelströmung
- 221: Mischzone
- 222: Plasmazone
- 223: Plasma
- 230: Verbindungsstück
- 231: Erster Abschnitt
- 232: Zweiter Abschnitt
- 233: Dritter Abschnitt
- 235: Erstes Eingangsgas
- 236: Zweites Eingangsgas
- 237: Drittes Eingangsgas
- 238: Produktstrom
- 239: Auslass
- 240: Zweiter Reaktor
- 242, 243: Zweite Endflächen
- 244: Durchmesser des Verbindungsstücks
- 245: Zweite Mantelfläche
- 246: Erster Reaktordurchmesser
- 247: Zweiter Reaktordurchmesser
- 248: Gesamtlänge
- 249: Konisches Ende
- 250: Innenrohr
- 251: Innerer Gasraum
- 252: Äußerer Gasraum
- 253: Offenes Ende
- 254: Geschlossenes Ende
- 260: Vibrationsvorrichtung
- 300: Erster Wärmetauscher
- 302: Zweiter Wärmetauscher
- 304: Stromnetz
- 306: Gasnetz
- 308: Erneuerbare Energie
- 310: Wasserstoff-Generator
- 312: Kohlenstoffspeicher
- 314: Hydrovergasung
- 316: Methanisierung
- 318: Elektrolyse
- 320: Gasspeicher
- 322: Kohlenstoffmonoxid

## Patentansprüche

1. Vorrichtung (4) zur Durchführung einer chemischen Reaktion in einem Plasma (223), wobei die Vorrichtung (4) eine Quelle zur Erzeugung elektromagnetischer Wellen (211), mindestens einen ersten Reaktor (200), mindestens ein Verbindungsstück (230) und einen zweiten Reaktor (240) umfasst,
wobei der erste Reaktor (200), das Verbindungsstück (230) und der zweite Reaktor (240) jeweils als Rohre ausgeführt sind, das Verbindungstück (230) einen kleineren Durchmesser (244) aufweist als der erste Reaktor (200) und die Quelle zur Erzeugung elektromagnetischer Wellen (211) an dem ersten Reaktor (200) angeordnet ist,
wobei der erste Reaktor (200) eine erste Mantelfläche (201) und erste Endflächen (213, 214) aufweist und der zweite Reaktor (240) eine zweite Mantelfläche (245) und zweite Endflächen (242, 243) aufweist und der zweite Reaktor (240) ein zumindest teilweise in dem zweiten Reaktor (240) angeordnetes Innenrohr (250) besitzt, so dass ein innerer Gasraum (251) und ein äußerer Gasraum (252) gebildet werden, die an der dem Verbindungsstück (230) näherliegenden zweiten Endfläche (243) des zweiten Reaktors (240) voneinander getrennt sind,
wobei das Verbindungstück (230) den ersten Reaktor (200) und den zweiten Reaktor (240) fluidisch miteinander verbindet und das Verbindungsstück (230) an einer der ersten Endflächen (214) aus dem ersten Reaktor (200) austritt und an der zweiten Mantelfläche (245) in den äußeren Gasraum (252) des zweiten Reaktors (240) mündet, insbesondere in tangentialer Ausrichtung, und wobei die erste Mantelfläche (201) des ersten Reaktors (200) einen ersten Abschnitt (231), mit mindestens einem Einlass (212) zur Zufuhr eines ersten Eingangsgases (235), einen zweiten Abschnitt (232) mit mindestens einem Einlass (212) zur Zufuhr eines zweiten Eingangsgases (236) und ein für die elektromagnetischen Wellen (211) durchlässiges Fenster (210), das insbesondere aus Quarz, Aluminiumoxid, Bornitrid oder Polytetrafluorethylen hergestellt ist, aufweist,
wobei der Einlass (212) zur Zufuhr des ersten Eingangsgases (235) und der Einlass (212) zur Zufuhr des zweiten Eingangsgases (236) tangential zur ersten Mantelfläche (201) des ersten Reaktors (200) ausgerichtet sind und der erste Abschnitt (231) und der zweite Abschnitt (232) axial versetzt angeordnet sind, wobei der zweite Abschnitt (232) näher als der erste Abschnitt (231) an der ersten Endfläche (214) liegt, aus der das Verbindungsstück (230) aus dem ersten Reaktor (200) austritt, und das Fenster (210) zwischen dem ersten Abschnitt (231) und dem zweiten Abschnitt (232) angeordnet ist und
der zweite Reaktor (240), insbesondere an einer der zweiten Endflächen (242, 243), einen Auslass (239) zur Abfuhr eines Produktstroms (238) aufweist und die zweite Mantelfläche (245) des zweiten Reaktors (240) einen dritten Abschnitt (233) mit mindestens einem Einlass (212) zur Zufuhr eines dritten Eingangsgases (237) aufweist und der dritte Abschnitt (233) insbesondere an einer dem Verbindungsstück (230) entfernteren der zweiten Endflächen (242) des zweiten Reaktors (240) angeordnet ist.

2. Vorrichtung (4) nach Anspruch 1, wobei sich das Verbindungsstück (230) in dem ersten Reaktor (200) über den mindestens einen Einlass (212) des zweiten Abschnitts (232) bis maximal zu dem Fenster (210) erstreckt.

3. Vorrichtung (4) nach einem der vorhergehenden Ansprüche, wobei der erste Reaktor (200) und das Verbindungsstück (230) koaxial angeordnet sind und/oder der zweite Reaktor (240) und das Innenrohr (250) koaxial angeordnet sind.

4. Vorrichtung (4) nach einem der vorhergehenden Ansprüche, wobei der erste Abschnitt (231) und/oder der zweite Abschnitt (232) jeweils mindestens zwei Einlässe (212) aufweisen und die mindestens zwei Einlässe (212) bevorzugt gegenüberliegend angeordnet sind, bezüglich einer Mittelachse des ersten Reaktors (200).

5. Vorrichtung (4) nach einem der vorhergehenden Ansprüche, wobei die Vorrichtung (4) mindestens zwei, insbesondere vier, sechs, acht oder zwölf, erste Reaktoren (200) mit jeweils einem Verbindungsstück (230) aufweist und insbesondere die Verbindungsstücke (230) jeweils tangential in den äußeren Gasraum (252) des zweiten Reaktors (240) münden.

6. Vorrichtung (4) nach einem der vorhergehenden Ansprüche, wobei das Verbindungsstück (230) einen konstanten Durchmesser (244) aufweist und gegebenenfalls der erste Reaktor (200) und/oder der zweite Reaktor (240) jeweils einen konstanten Reaktordurchmesser (246, 247) aufweisen.

7. Vorrichtung (4) nach einem der vorhergehenden Ansprüche, wobei das Innenrohr (250) ein offenes Ende (253) und ein geschlossenes Ende (254) aufweist und das offenen Ende (253) an dem Auslass (239) des zweiten Reaktors (240) angeordnet ist oder das Innenrohr (250) zwei offene Enden (253) aufweist und eines der zwei offenen Enden (253) den Auslass (239) des zweiten Reaktors (240) bildet.

8. Vorrichtung (4) nach einem der vorhergehenden Ansprüche, wobei der zweite Reaktor (240) ein konisches Ende aufweist und der Auslass am konischen Ende (249) angeordnet ist.

9. Vorrichtung (4) nach einem der vorhergehenden Ansprüche, wobei das Verbindungsstück (230) und/oder das Innenrohr (250) mit einer Vibrationsvorrichtung (260) ausgerüstet sind und/oder dass zumindest Teile der ersten Mantelfläche (201) des ersten Reaktors (200) und/oder das Verbindungsstück (230) mit einer Heizvorrichtung ausgerüstet sind.

10. Vorrichtung (4) nach einem der vorhergehenden Ansprüche, wobei dass der erste Reaktor (200) aus einem für die elektromagnetischen Wellen (211) undurchlässigem Material wie Stahl, Bronze oder Aluminium und/oder das Verbindungsstück (230) aus einem hochtemperaturstabilen Material wie Graphit, Quarzglas oder einem Metall wie Wolfram oder Molybdän hergestellt ist.

11. Vorrichtung (4) nach einem der vorhergehenden Ansprüche, wobei sich das Innenrohr (250) von der dem Verbindungstück (230) näherliegenden zweiten Endfläche (243) des zweiten Reaktors (240) bis zu einer Länge in einem Bereich von 50% bis 70% einer Gesamtlänge (248) des zweiten Reaktors (240) erstreckt.

12. Verfahren zur Durchführung der chemischen Reaktion unter Verwendung der Vorrichtung (4) nach einem der Ansprüche 1 bis 12, wobei ein Feststoff, insbesondere Kohlenstoff (106), beteiligt ist, insbesondere gebildet oder umgesetzt wird, und die chemische Reaktion bevorzugt ausgewählt ist aus der Gruppe bestehend aus Pyrolyse von Kohlenwasserstoffen, insbesondere von Methan (103), Herstellung von Acetylen, Reformierung von Kohlenwasserstoffen, insbesondere Methan (103), Pyrolyse von Schwefelwasserstoff, Pyrolyse von Ammoniak und Hydrovergasung von Kohlenstoff.

13. Verfahren nach Anspruch 12, wobei das erste Eingangsgas (235), insbesondere enthaltend Methan (103), an dem mindestens einen Einlass (212) des ersten Abschnitts (231) und das zweite Eingangsgas (236), insbesondere enthaltend ein Inertgas, Wasserdampf und/oder Wasserstoff (110), an dem mindestens einen Einlass (212) des zweiten Abschnitts (232) dem ersten Reaktor (200) zugeführt werden und ein Produktstrom (238), insbesondere enthaltend den Feststoff, an dem Auslass (239) dem zweiten Reaktor (240) entnommen wird, wobei der erste Abschnitt (231) auf einer dem Verbindungsstück (230) gegenüberliegenden Seite des Fensters (210) angeordnet ist und
wobei das erste Eingangsgas (235) und das zweite Eingangsgas (236) jeweils tangential in den ersten Reaktor (200) geführt werden, in dem ersten Reaktor (200) gemischt werden und unter Einwirkung der elektromagnetischen Wellen (211) ein Plasma (223) bilden, insbesondere in dieser Reihenfolge, wobei das gebildete Plasma (223) vorzugsweise tangential in den zweiten Reaktor (240) geführt wird.

14. Verfahren nach einem der Ansprüche 12 bis 13, wobei der Produktstrom zumindest teilweise zurückgeführt wird, insbesondere in den ersten Reaktor.

15. Verfahren nach einem der Ansprüche 12 bis 14, wobei Wärme von dem Produktstrom auf das erste Eingangsgas und/oder das zweite Eingangsgas übertragen wird.

## Claims

1. Apparatus (4) for conducting a chemical reaction in a plasma (223), wherein the apparatus (4) comprises a source for generation of electromagnetic waves (211), at least one first reactor (200), at least one connecting piece (230) and a second reactor (240),
wherein the first reactor (200), the connecting piece (230) and the second reactor (240) are each designed as tubes, the connecting piece (230) has a smaller diameter (244) than the first reactor (200), and the source for generation of electromagnetic waves (211) is disposed on the first reactor (200),
wherein the first reactor (200) has a first outer face (201) and first end faces (213, 214), and the second reactor (240) has a second outer face (245) and second end faces (242, 243), and the second reactor (240) has an inner tube (250) disposed at least partly within the second reactor (240), so as to form an inner gas space (251) and an outer gas space (252) that are separated from one another at the second end face (243) of the second reactor (240) that is closer to the connecting piece (230),
wherein the connecting piece (230) fluidically interconnects the first reactor (200) and the second reactor (240), and the connecting piece (230) exits from the first reactor (200) at one of the first end faces (214) and opens into the outer gas space (252) of the second reactor (240) at the second outer face (245), especially in tangential direction, and
wherein the first outer face (201) of the first reactor (200) has a first section (231) having at least one inlet (212) for supply of a first input gas (235), a second section (232) having at least one inlet (212) for supply of a second input gas (236), and a window (210) which is transparent to the electromagnetic waves (211) and has been produced in particular from quartz, alumina, boron nitride or polytetrafluoroethylene,
wherein the inlet (212) for supply of the first input gas (235) and the inlet (212) for supply of the second input gas (236) are aligned tangentially to the first outer face (201) of the first reactor (200) and
the first section (231) and the second section (232) are in an axially offset arrangement, wherein the second section (232) lies closer than the first section (231) to the first end face (214) from which the connecting piece (230) exits from the first reactor (200), and the window (210) is disposed between the first section (231) and the second section (232) and
the second reactor (240), particularly at one of the second end faces (242, 243), has an outlet (239) for removal of a product stream (238) and
the second outer face (245) of the second reactor (240) has a third section (233) having at least one inlet (212) for supply of a third input gas (237), and the third section (233) is especially disposed at one of the second end faces (242) of the second reactor (240) that is further removed from the connecting piece (230).

2. Apparatus (4) according to Claim 1, wherein the connecting piece (230) extends within the first reactor (200) via the at least one inlet (212) of the second section (232) up to no further than the window (210).

3. Apparatus (4) according to either of the preceding claims, wherein the first reactor (200) and the connecting piece (230) are in a coaxial arrangement and/or the second reactor (240) and the inner tube (250) are in a coaxial arrangement.

4. Apparatus (4) according to any of the preceding claims, wherein the first section (231) and/or the second section (232) each have at least two inlets (212), and the at least two inlets (212) are preferably arranged opposite one another, with respect to a centre axis of the first reactor (200).

5. Apparatus (4) according to any of the preceding claims, wherein the apparatus (4) has at least two, especially four, six, eight or twelve, first reactors (200) each having a connecting piece (230), and, in particular, the connecting pieces (230) each open tangentially into the outer gas space (252) of the second reactor (240).

6. Apparatus (4) according to any of the preceding claims, wherein the connecting piece (230) has a constant diameter (244), and the first reactor (200) and/or the second reactor (240) optionally each have a constant reactor diameter (246, 247).

7. Apparatus (4) according to any of the preceding claims, wherein the inner tube (250) has an open end (253) and a closed end (254), and the open end (253) is disposed at the outlet (239) of the second reactor (240) or the inner tube (250) has two open ends (253), and one of the two open ends (253) forms the outlet (239) of the second reactor (240).

8. Apparatus (4) according to any of the preceding claims, wherein the second reactor (240) has a conical end and the outlet is disposed at the conical end (249).

9. Apparatus (4) according to any of the preceding claims, wherein the connecting piece (230) and/or the inner tube (250) are equipped with a vibration apparatus (260) and/or that at least parts of the first outer face (201) of the first reactor (200) and/or the connecting piece (230) are equipped with a heating apparatus.

10. Apparatus (4) according to any of the preceding claims, wherein the first reactor (200) has been manufactured from a material impervious to the electromagnetic waves (211), such as steel, bronze or aluminium, and/or the connecting piece (230) has been manufactured from a material of high thermal stability, such as graphite, quartz glass or a metal such as tungsten or molybdenum.

11. Apparatus (4) according to any of the preceding claims, wherein the inner tube (250) of the second end face (243) of the second reactor (240) that is closer to the connecting piece (230) extends up to a length within a range from 50% to 70% of a total length (248) of the second reactor (240).

12. Method of conducting the chemical reaction using the apparatus (4) according to any of Claims 1 to 12, wherein a solid material, especially carbon (106), is involved and is especially formed or converted, and the chemical reaction is preferably selected from the group consisting of pyrolysis of hydrocarbons, especially of methane (103), production of acetylene, reforming of hydrocarbons, especially methane (103), pyrolysis of hydrogen sulfide, pyrolysis of ammonia and hydrogasification of carbon.

13. Method according to Claim 12, wherein the first input gas (235), especially comprising methane (103), is fed to the first reactor (200) at the at least one inlet (212) of the first section (231), and the second input gas (236), especially comprising an inert gas, water vapour and/or hydrogen (110), at the at least one inlet (212) of the second section (232), and a product stream (238), especially comprising the solid material, is withdrawn from the second reactor (240) at the outlet (239), wherein the first section (231) is disposed on an opposite side of the window (210) from the connecting piece (230), and
wherein the first input gas (235) and the second input gas (236) are each conducted tangentially into the first reactor (200) and mixed in the first reactor (200), and form a plasma (223) under the action of the electromagnetic waves (211), especially in that sequence, wherein the plasma (223) formed is conducted preferably tangentially into the second reactor (240).

14. Method according to any of Claims 12 to 13, wherein the product stream is at least partly recycled, especially into the first reactor.

15. Method according to any of Claims 12 to 14, wherein heat is transferred from the product stream to the first input gas and/or the second input gas.

## Revendications

1. Dispositif (4) pour réaliser une réaction chimique dans un plasma (223), le dispositif (4) comprenant une source destinée à générer des ondes électromagnétiques (211), au moins un premier réacteur (200), au moins une pièce de raccordement (230) et un deuxième réacteur (240),
le premier réacteur (200), la pièce de raccordement (230) et le deuxième réacteur (240) étant respectivement réalisés sous la forme de tubes, la pièce de raccordement (230) présentant un diamètre (244) inférieur à celui du premier réacteur (200) et la source destinée à générer des ondes électromagnétiques (211) étant disposée au niveau du premier réacteur (200),
le premier réacteur (200) présentant une première surface latérale (201) et des premières faces d'extrémité (213, 214) et le deuxième réacteur (240) présentant une deuxième surface latérale (245) et des deuxièmes faces d'extrémité (242, 243) et le deuxième réacteur (240) possédant un tube intérieur (250) disposé au moins partiellement dans le deuxième réacteur (240), de manière à former un espace gazeux intérieur (251) et un espace gazeux extérieur (252) séparés l'un de l'autre au niveau de la deuxième face d'extrémité (243) du deuxième réacteur (240), plus près de la pièce de raccordement (230),
la pièce de raccordement (230) reliant fluidiquement l'un à l'autre le premier réacteur (200) et le deuxième réacteur (240) et la pièce de raccordement (230) sortant du premier réacteur (200) au niveau de l'une des premières faces d'extrémité (214) et débouchant dans l'espace gazeux extérieur (252) du deuxième réacteur (240) au niveau de la deuxième face latérale (245), en particulier selon une orientation tangentielle, et
la première surface latérale (201) du premier réacteur (200) comprenant une première partie (231) présentant au moins une entrée (212) pour l'apport d'un premier gaz d'entrée (235), une deuxième partie (232) présentant au moins une entrée (212) pour l'apport d'un deuxième gaz d'entrée (236) et une fenêtre (210) perméable aux ondes électromagnétiques (211), laquelle est produite en particulier à partir de quartz, d'oxyde d'aluminium, de nitrure de bore ou de polytétrafluoroéthylène,
l'entrée (212) destinée à l'apport du premier gaz d'entrée (235) et l'entrée (212) destinée à l'apport du deuxième gaz d'entrée (236) étant orientées tangentiellement à la première surface latérale (201) du premier réacteur (200), et
la première partie (231) et la deuxième partie (232) étant disposées de manière décalée axialement, la deuxième partie (232) étant plus proche que la première partie (231) de la première face d'extrémité (214) d'où sort la pièce de raccordement (230) du premier réacteur (200), et la fenêtre (210) étant disposée entre la première partie (231) et la deuxième partie (232), et le deuxième réacteur (240) présentant, en particulier au niveau de l'une des deuxièmes faces d'extrémité (242, 243), une sortie (239) destinée à l'évacuation d'un courant de produit (238), et
la deuxième surface latérale (245) du deuxième réacteur (240) comportant une troisième partie (233) présentant au moins une entrée (212) pour l'apport d'un troisième gaz d'entrée (237), et la troisième partie (233) étant disposée en particulier au niveau de celle des deuxièmes faces d'extrémité (242) du deuxième réacteur (240) qui est la plus éloignée de la pièce de raccordement (230).

2. Dispositif (4) selon la revendication 1, dans lequel la pièce de raccordement (230) s'étend dans le premier réacteur (200) par-delà l'au moins une entrée (212) de la deuxième partie (232) au maximum jusqu'à la fenêtre (210).

3. Dispositif (4) selon l'une quelconque des revendications précédentes, dans lequel le premier réacteur (200) et la pièce de raccordement (230) sont disposés coaxialement et/ou le deuxième réacteur (240) et le tube intérieur (250) sont disposés coaxialement.

4. Dispositif (4) selon l'une quelconque des revendications précédentes, dans lequel la première partie (231) et/ou la deuxième partie (232) présentent respectivement au moins deux entrées (212) et les au moins deux entrées (212) sont de préférence disposées en vis-à-vis l'une de l'autre par rapport à un axe central du premier réacteur (200).

5. Dispositif (4) selon l'une quelconque des revendications précédentes, dans lequel le dispositif (4) comporte au moins deux, en particulier quatre, six, huit ou douze premiers réacteurs (200) respectivement dotés d'une pièce de raccordement (230), et en particulier les pièces de raccordement (230) débouchent respectivement de manière tangentielle dans l'espace gazeux extérieur (252) du deuxième réacteur (240).

6. Dispositif (4) selon l'une quelconque des revendications précédentes, dans lequel la pièce de raccordement (230) présente un diamètre constant (244) et, éventuellement, le premier réacteur (200) et/ou le deuxième réacteur (240) présentent respectivement un diamètre de réacteur constant (246, 247).

7. Dispositif (4) selon l'une quelconque des revendications précédentes, dans lequel le tube intérieur (250) présente une extrémité ouverte (253) et une extrémité fermée (254), et l'extrémité ouverte (253) est disposée au niveau de la sortie (239) du deuxième réacteur (240), ou
le tube intérieur (250) présente deux extrémités ouvertes (253) et l'une des deux extrémités ouvertes (253) constitue la sortie (239) du deuxième réacteur (240).

8. Dispositif (4) selon l'une quelconque des revendications précédentes, dans lequel le deuxième réacteur (240) présente une extrémité conique et la sortie est disposée à l'extrémité conique (249).

9. Dispositif (4) selon l'une quelconque des revendications précédentes, dans lequel la pièce de raccordement (230) et/ou le tube intérieur (250) sont équipés d'un dispositif vibrant (260) et/ou en ce qu'au moins des parties de la première surface latérale (201) du premier réacteur (200) et/ou de la pièce de raccordement (230) sont équipées d'un dispositif de chauffage.

10. Dispositif (4) selon l'une quelconque des revendications précédentes, dans lequel le premier réacteur (200) est produit en un matériau imperméable aux ondes électromagnétiques (211), tel que l'acier, le bronze ou l'aluminium, et/ou la pièce de raccordement (230) est produite en un matériau résistant aux hautes températures, tel que le graphite, le verre quartzeux ou un métal tel que le tungstène ou le molybdène.

11. Dispositif (4) selon l'une quelconque des revendications précédentes, dans lequel le tube intérieur (250) s'étend à partir de la deuxième face d'extrémité (243) du deuxième réacteur (240), la plus proche de la pièce de raccordement (230), sur une longueur comprise dans une plage allant de 50 % à 70 % d'une longueur totale (248) du deuxième réacteur (240).

12. Procédé pour la mise en œuvre de la réaction chimique par utilisation du dispositif (4) selon l'une quelconque des revendications 1 à 12, dans lequel un solide, en particulier du carbone (106), est impliqué, en particulier formé ou mis à réagir, et la réaction chimique est de préférence choisie dans le groupe constitué par la pyrolyse d'hydrocarbures, en particulier de méthane (103), la préparation d'acétylène, le reformage d'hydrocarbures, en particulier de méthane (103), la pyrolyse du sulfure d'hydrogène, la pyrolyse de l'ammoniac et l'hydrogazéification du carbone.

13. Procédé selon la revendication 12, dans lequel le premier gaz d'entrée (235), contenant en particulier du méthane (103), au niveau de l'au moins une entrée (212) de la première partie (231) et le deuxième gaz d'entrée (236), contenant en particulier un gaz inerte, de la vapeur d'eau et/ou de l'hydrogène (110), au niveau de l'au moins une entrée (212) de la deuxième partie (232) sont amenés au premier réacteur (200), et un courant de produit (238), contenant en particulier le solide, est soutiré du deuxième réacteur (240) au niveau de la sortie (239), la première partie (231) étant disposée sur un côté de la fenêtre (210) qui est opposé à la pièce de raccordement (230), et
dans lequel le premier gaz d'entrée (235) et le deuxième gaz d'entrée (236) sont respectivement acheminés tangentiellement dans le premier réacteur (200), mélangés dans le premier réacteur (200) et forment un plasma (223) sous l'action des ondes électromagnétiques (211), en particulier dans cet ordre, le plasma (223) formé est de préférence acheminé tangentiellement dans le deuxième réacteur (240).

14. Procédé selon l'une quelconque des revendications 12 à 13, dans lequel le courant de produit est recyclé au moins partiellement, en particulier dans le premier réacteur.

15. Procédé selon l'une quelconque des revendications 12 à 14, dans lequel de la chaleur est transférée du courant de produit au premier gaz d'entrée et/ou au deuxième gaz d'entrée.
